# EUROPEAN PATENT SPECIFICATION

(11) **EP 1 881 075 B1**
(45) Date of publication and mention of the grant of the patent: **24.07.2013**
(21) Application number: 07011311.3
(22) Date of filing: 09.05.1995
(51) Int. Cl.: C12N 15/86, C12N 5/10, C12N 7/04, C12N 9/00, C07K 14/00, C07K 14/005, C07K 14/15, C07K 14/54

(54) **Retroviral vectors having a reduced recombination rate**
Retrovirale Vektoren mit reduzierter Rekombinationsrate
Vecteurs rétroviraux dotés d'un taux réduit de recombinaisons

(30) Priority: 09.05.1994 US 240030
(43) Date of publication of application: 23.01.2008
(62) Divisional of application: 95920386.0
(73) Proprietor: Oxford BioMedica (UK) Limited, The Oxford Science Park Oxford OX4 4GA (GB)
(72) Inventor: Respess, James G., San Diego, CA 92109 (US)
(74) Representative: Mallalieu, Catherine Louise

(56) References cited:
- WO-A-89/07150
- WO-A-90/02806
- WO-A-92/07943
- WO-A-94/29438
- TCHENIO T ET AL: "HIGH-FREQUENCY INTRACELLULAR TRANSPOSITION OF A DEFECTIVE MAMMALIAN PROVIRUS DETECTED BY AN IN-SITU COLORIMETRIC ASSAY" JOURNAL OF VIROLOGY, vol. 66, no. 3, 1992, pages 1571-1578, XP002459330 ISSN: 0022-538X
- MARKOWITZ D ET AL: "CONSTRUCTION AND USE OF A SAFE AND EFFICIENT AMPHOTROPIC PACKAGING CELL LINE" VIROLOGY, RAVEN PRESS, NEW YORK, NY, US, vol. 167, no. 2, December 1988 (1988-12), pages 400-406, XP000570006 ISSN: 0042-6822
- PAROLIN C ET AL: "ANALYSIS IN HUMAN IMMUNODEFICIENCY VIRUS TYPE 1 VECTORS OF CIS-ACTING SEQUENCES THAT AFFECT GENE TRANSFER INTO HUMAN LYMPHOCYTES" JOURNAL OF VIROLOGY, THE AMERICAN SOCIETY FOR MICROBIOLOGY, US, vol. 68, no. 6, 1994, pages 3888-3895, XP000915678 ISSN: 0022-538X
- DANOS O ET AL: "SAFE AND EFFICIENT GENERATION OF RECOMBINANT RETROVIRUSES WITH AMPHOTROPIC AND ECOTROPIC HOST RANGES" PROCEEDINGS OF THE NATIONAL ACADEMY OF SCIENCES OF USA, NATIONAL ACADEMY OF SCIENCE, WASHINGTON, DC, US, vol. 85, no. 17, 1 September 1988 (1988-09-01), pages 6460-6464, XP000569693 ISSN: 0027-8424
- NEUBAUER V ET AL: "CONSTRUCTION OF A RETROVIRAL VECTOR FOR INTRACELLULAR IMMUNIZATION WITH A HIV ANTISENSE SEQUENCE" VIII INTERNATIONAL CONFERENCE ON AIDS AND THE III STD WORLD CONGRESS. VIII INTERNATIONAL CONFERENCE ON AIDS AND THE III STD WORLD CONGRESS; HARVARD-AMSTERDAM CONFERENCE, AMSTERDAM, NETHERLANDS, JULY 19-24, 1992. PAGINATION VARIES VIII INTERNATIONAL C, 1992, page A82, XP002459331
- PRICE J ET AL: "LINEAGE ANALYSIS IN THE VERTEBRATE NERVOUS SYSTEM BY RETROVIRUS-MEDIATED GENE TRANSFER" PROCEEDINGS OF THE NATIONAL ACADEMY OF SCIENCES OF USA, NATIONAL ACADEMY OF SCIENCE, WASHINGTON, DC, US, vol. 84, no. 1, 1987, pages 156-160, XP002406390 ISSN: 0027-8424

## Description

### Technical Field

The present invention relates generally to a packaging cell line and a producer cell line for methods for producing retroviral vectors for use in gene transfer, and more specifically, to retroviral vectors which are constructed such that the formation of replication competent virus by recombination is precluded.

### Background of the Invention

Retroviruses are RNA viruses which can replicate and integrate into a host cell's genome through a DNA intermediate. This DNA intermediate, or provirus, may be stably integrated into the host's cellular DNA. Retroviruses are known to be responsible for a wide variety of diseases in both man and animals, including for example AIDS and a wide variety of cancers.

Although retroviruses can cause disease, they also have a number of properties that lead them to be considered as one of the most promising techniques for genetic therapy of disease. These properties include: (1) efficient entry of genetic material (the vector genome) into cells; (2) an active efficient process of entry into the target cell nucleus; (3) relatively high levels of gene expression; (4) minimal pathological effects on target cells; and (5) the potential to target particular cellular subtypes through control of the vector-target cell binding and tissue-specific control of gene expression. In using a retrovirus for genetic therapy, a foreign gene of interest may be incorporated into the retrovirus in place of normal retroviral RNA. When the retrovirus injects its RNA into a cell, the foreign gene is also introduced into the cell, and may then be integrated into the host's cellular DNA as if it were the retrovirus itself. Expression of this foreign gene within the host results in expression of foreign protein by the host cell.

Most retroviral vector systems which have been developed for gene therapy are based on murine retroviruses. Briefly, these retroviruses exist in two forms, as proviruses integrated into a host's cellular DNA, or as free virions. The virion form of the virus contains the structural and enzymatic proteins of the retrovirus (including reverse transcriptase), two RNA copies of the viral genome, and portions of the cell's plasma membrane in which is embedded the viral envelope glycoprotein. The genome is organized into four main regions: the Long Terminal Repeat (LTR), and the *gag, pol,* and *env* genes. The LTR may be found at both ends of the proviral genome, is a composite of the 5' and 3' ends of the RNA genome, and contains *cis*-acting elements necessary for the initiation and termination of transcription. The three genes *gag, pol,* and *env* are located between the terminal LTRs. The *gag* and *pol* genes encode, respectively, internal viral structures and enzymatic proteins (such as integrase). The *env* gene encodes the envelope glycoprotein (designated gp70 and p15e) which confers infectivity and host range specificity of the virus, as well as the "R" peptide of undetermined function.

An important consideration in using retroviruses for gene therapy is the availability of "safe" retroviruses. Packaging cell lines and vector producing cell lines have been developed to meet this concern. Briefly, this methodology employs the use of two components, a retroviral vector and a packaging cell line (PCL) The retroviral vector contains long terminal repeats (LTRs), the foreign DNA to be transferred and a packaging sequence (ψ). This retroviral vector will not reproduce by itself because the genes which encode structural and envelope proteins are not included within the vector genome. The PCL contains genes encoding the *gag, pol,* and *env* proteins, but does not contain the packaging signal "ψ". Thus, a PCL can only form empty virion particles by itself. Within this general method, the retroviral vector is introduced into the PCL, thereby creating a vector-producing cell line (VCL). This VCL manufactures virion particles containing only the retroviral vector's (foreign) genome, and therefore has previously been considered to be a safe retrovirus vector for therapeutic use.

There are, however, several shortcomings with the current use of VCLs. One issue involves the generation of "live virus" (*i.e*., replication competent retrovirus; RCR) by the VCL. Briefly, RCR can be produced in conventional producer cells when: (1) The vector genome and the helper genomes recombine with each other; (2) The vector genome or helper genome recombines with homologous cryptic endogenous retroviral elements in the producer cell; or (3) Cryptic endogenous retroviral elements reactivate (*e.g*., xenotropic retroviruses in mouse cells).

Another issue is the propensity of mouse based VCLs to package endogenous retrovirus-like elements (which can contain oncogenic gene sequences) at efficiencies close to that with which they package the desired retroviral vector. Such elements, because of their retrovirus-like structure, are transmitted to the target cell to be treated at frequencies that parallel its transfer of the desired retroviral vector sequence.

A third issue is the ability to make sufficient retroviral vector particles at a suitable concentration to: (1) treat a large number of cells (*e.g*., 10⁸ - 10¹⁰); and (2) manufacture vector particles at a commercially viable cost.

In order to construct safer PCLs, researchers have generated deletions of the 5' LTR and portions of the 3' LTR of helper elements (*see,* Miller and Buttimore, Mol. Cell. Biol. 6:2895-2902, 1986). When such cells are used, two recombination events are necessary to form the wild-type, replication competent genome. Nevertheless, results from several laboratories have indicated that even when several deletions are present, RCR may still be generated (*see*, Bosselman et al., Mol. Cell. Biol. 7:1797-1806, 1987; Danos and Mulligan, Proc. Nat'l. Acad. Sci. USA 81:6460-6464, 1988). In addition, cell lines containing both 5' and 3' LTR deletions which have been constructed have thus far not proven useful since they produce relatively low titers (Dougherty et al., J. Virol. 63:3209-3212, 1989).

One of the more recent approaches to constructing safer packaging cell lines involves the use of complementary portions of helper virus elements, divided among two separate plasmids, one containing *gag* and *pol*, and the other containing *env* (see, Markowitz et al., J Virol. 62:1120-1124; and Markowitz et al., Virology 167:600-606, 1988. One benefit of this double-plasmid system is that three recombination events are required to generate a replication competent genome. Nevertheless, these double-plasmid vectors have also suffered from the drawback of including portions of the retroviral LTRs, and therefore remain capable of producing infectious virus.

The present invention overcomes the difficulties of recombination and low titer associated with many of the prior packaging cell lines, and further provides other related advantages.

### Summary of the Invention

According to one aspect of the present invention there is provided a packaging cell line, comprising a *gag*/*pol* expression cassette comprising a promoter operably linked to a *gag*/*pol* gene, and a polyadenylation sequence, wherein the 5' terminal end of said *gag*/*pol* gene has been modified to contain at least 25 codons which are degenerate for *gag* and an *env* expression cassette.

According to another aspect of the invention there is provided a producer cell line comprising a packaging cell line of the present invention and a retrovirus vector construct.

According to another aspect of the present invention there is provided a method of producing a packaging cell, comprising:
(a) introducing a *gag*/*pol* expression cassette of the present invention into an animal cell;
(b) selecting a cell containing a *gag*/*pol* expression cassette which expresses high levels of *gag*/*pol;*
(c) introducing an *env* expression cassette into said selected cell; and
(d) selecting a cell which expresses high levels of *env*, and thereby producing said packaging cell.

According to another aspect of the present invention there is provided a method of producing recombinant retroviral particles, comprising introducing a retroviral vector construct into packaging cell line produced by a method according to the present invention.

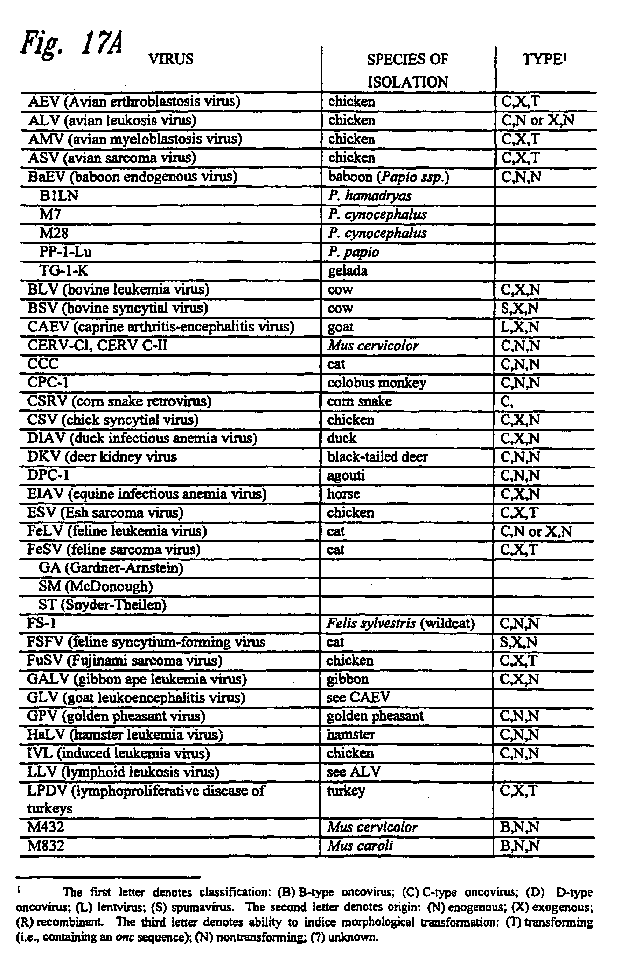

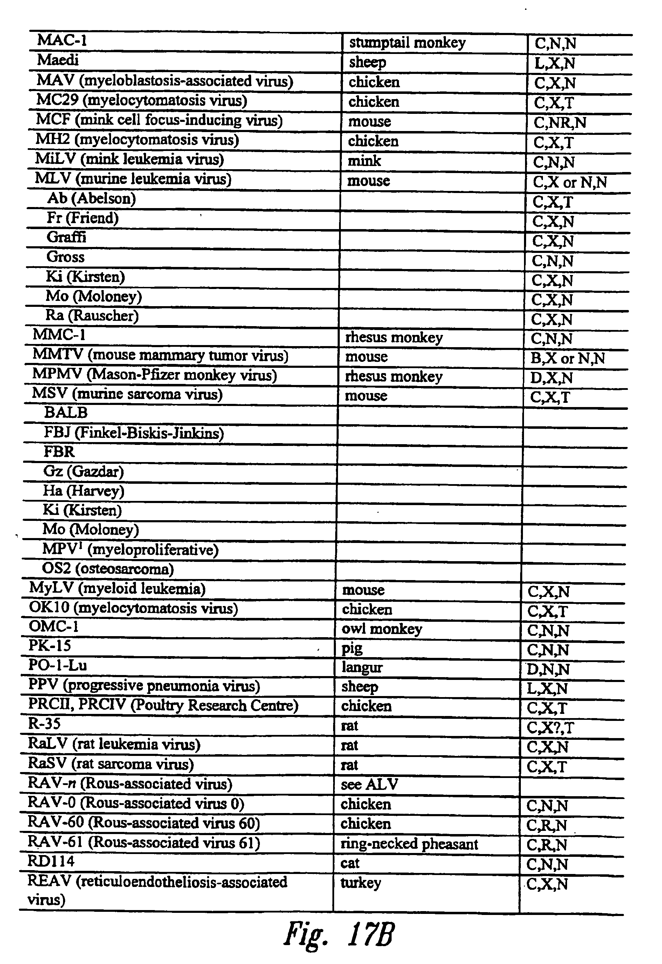

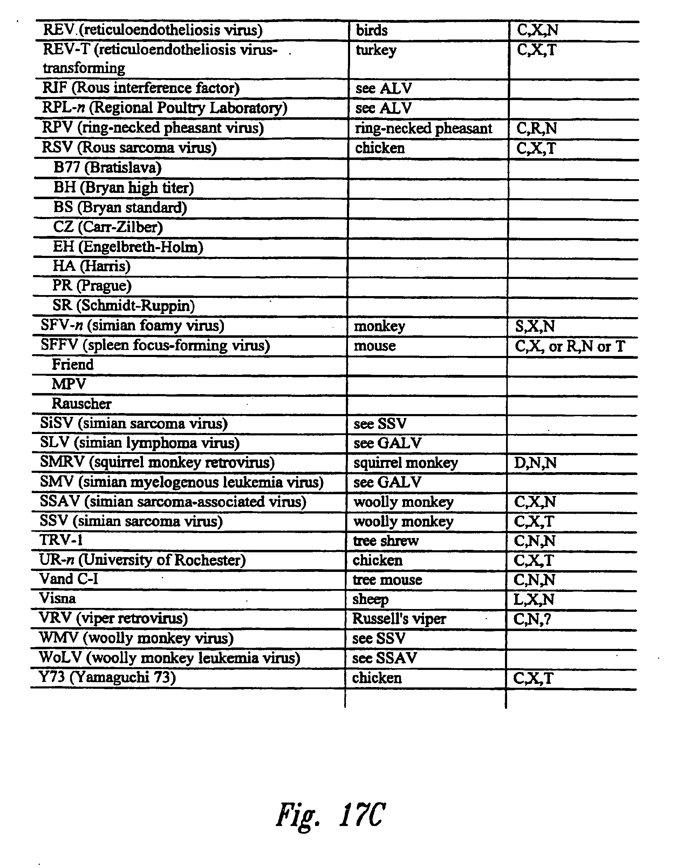

Briefly stated, the present invention provides compositions and methods for the construction of packaging cell lines which, preclude the formation of RCR by homologous recombination. We describe recombinant retroviral vector constructs (RETROVECTOR^{™}) comprising a 5' LTR, a tRNA binding site, a packaging signal, one or more heterologous sequences, an origin of second strand DNA synthesis, and a 3' LTR, wherein the retroviral vector construct lacks *gag*/*pol* and *env* coding sequences. According to the invention, the retroviral vector construct lacks an extended packaging signal. Within one embodiment, the retroviral vector construct lacks a retroviral nucleic acid sequence upstream of the 5' LTR. Within a preferred embodiment, the retroviral vector constructs lack an *env* coding sequence upstream of the 5' LTR. Retroviral vector constructs described herein may be constructed from one or more retroviruses, including, for example, a wide variety of amphotropic, ecotropic, xenotropic, and polytropic viruses (*see e.g.,* Figures 17A, B, and C).

As noted above, retroviral vector constructs described herein include one or more heterologous sequences. The heterologous sequence may be at least x kb in length, wherein x is selected from the group consisting of 1, 2, 3, 4, 5, 6, 7 and 8. The heterologous sequence may be a gene encoding a cytotoxic protein, such as, for example, ricin, abrin, diphtheria toxin, cholera toxin, gelonin, pokeweed, antiviral protein, tritin, Shigella toxin, and Pseudomonas exotoxin A. The heterologous sequence may be an antisense sequence, or an immune accessory molecule. Representative examples of immune accessory molecules include IL-1, IL-3, IL-4, IL-5, IL-6, IL-7, IL-8, IL-9, IL-10, IL-11, IL-13, and IL-14. Particularly preferred immune accessory molecules may be selected from the group consisting of IL-2, IL-12, IL-15 and gamma-interferon, or the group consisting of ICAM-1, ICAM-2, β-microglobin, LFA3, HLA class I and HLA class II molecules.

The heterologous sequence may encode a gene product that activates a compound with little or no cytotoxicity into a toxic product. Representative examples of such gene products include type I thymidine kinases such as HSVTK and VZVTK. The heterologous sequence may be a ribozyme. The heterologous sequence may be a replacement gene, which encode proteins such as Factor VIII, ADA, HPRT, CF and the LDL Receptor. The heterologous sequence may encode an immunogenic portion of a virus selected from the group consisting of HBC, HCV, HPV, EBV, FeLV, FIV, and HIV.

We describe *gag*/*pol* expression comprising a promoter operably linked to a *gag*/*pol* gene, and a polyadenylation sequence, wherein the *gaglpol* gene has been modified to contain codons which are degenerate for gag. Within one embodiment, the 5' terminal end of the *gag*/*pol* gene lacks a retroviral packaging signal sequence. Within other aspects *gag*/*pol* expression cassettes are used comprising a promoter operably linked to a *gag*/*pol* gene, and a polyadenylation sequence, wherein the expression cassette does not co-encapsidate with a replication competent virus.

Within other embodiments of the present invention, *gag*/*pol* expression cassettes are used comprising a promoter operably linked to a *gag*/*pol* gene, and a polyadenylation sequence, wherein a 3' terminal end of the *gag*/*pol* gene has been deleted without effecting the biological activity of integrase. Within one embodiment, a 5' terminal end of the *gag*/*pol* gene has been modified to contain codons which are degenerate for gag. Within a further embodiment, the 5' terminal end of the *gag*/*pol* gene lacks a retroviral packaging signal sequence. Within other embodiments, the 3' terminal end has been deleted upstream (5') of nucleotide 5751 of SEQ ID NO: 1.

Within other embodiments of the present invention, *env* expression cassettes are used, comprising a promoter operably linked to an *env* gene, and a polyadenylation sequence, wherein no more than 6 retroviral nucleotides are included upstream of the *env* gene. Within another embodiment, *env* expression cassettes are used comprising a promoter operably linked to an *env* gene, and a polyadenylation sequence, wherein the *env* expression cassette does not contain a consecutive sequence of more than 8 nucleotides which are found in a *gag*/*pol* gene. Within yet another embodiment, *env* expression cassettes are used comprising a promoter operably linked to an *env* gene, and a polyadenylation sequence, wherein a 3' terminal end of the *env* gene has been deleted without effecting the biological activity of env. Within one embodiment, the 3' terminal end of the gene has been deleted such that a complete R peptide is not produced by the expression cassette. Within a further embodiment, the e*nv* gene is derived from a type C retrovirus, and the 3' terminal end has been deleted such that the *en*v gene includes less than 18 nucleic acids which encode the R peptide. Within a preferred embodiment, the 3' terminal end has been deleted downstream from nucleotide 7748 of SEQ ID NO: 1.

Within various embodiments of the invention, the promoters of the *gag*/*pol* and *env* expression cassettes described above are heterologous promoters, such as CMV IE, the HVTK promoter, RSV promoter, Adenovirus major-later promoter and the SV40 promoter. Within other embodiments, the polyadenylation sequence is a heterologous polyadenylation sequence, such as the SV40 late poly A Signal and the SV40 early poly A Signal.

Within another embodiment of the present invention, packaging cell lines are provided, comprising a *gag*/*pol* expression cassette and an *env* expression cassette, wherein the *gag*/*pol* expression cassette lacks a consecutive sequence of greater than 20, preferably greater than 15, more preferably greater than 10, and most preferably greater than 8 consecutive nucleotides which are found in the *env* expression cassette. Within other embodiments, producer cell lines are provided comprising a *gag*/*pol* expression cassette, *env* expression cassette, and a retroviral vector construct, wherein the *gag*/*pol* expression cassette, *env* expression cassette and retroviral vector construct lack a consecutive sequence of greater than 20, preferably greater than 15, more preferably greater than 10, and most preferably greater than 8 nucleotides in common. Representative examples of such retroviral vector constructs, *gag*/*pol* and *env* expression cassettes are described in more detail below.

Within yet another aspect of the present invention, producer cell lines are provided comprising a packaging cell line as described above, and a retroviral vector construct. Within another embodiment of the present invention, producer cell lines are provided comprising a *gaglpol* expression cassette, *env* expression cassette and a retroviral vector construct, wherein the *gag*/*pol* expression cassette, *env* expression cassette and retroviral vector construct lack a consecutive sequence of greater than eight nucleotides in common.

Within other aspects of the invention, methods of producing a packaging cell line are provided, comprising the steps of (a) introducing a *gag*/*pol* expression cassette as described above into an animal cell; (b) selecting a cell containing a *gag*/*pol* expression cassette which expresses high levels of *gag*/*pol,* (c) introducing an *env* expression cassette into said selected cell, and (d) selecting a cell which expresses high levels of *env* and thereby producing the packaging cell. Within other embodiments of the invention, the *env* expression cassette may be introduced into the cell first, followed by the *gag*/*pol* expression cassette. Within other aspects, methods are provided for producing recombinant retroviral particles comprising the step of introducing a retroviral vector construct into a packaging cell as described above. Within preferred embodiments, the retroviral vector construct is one of the retroviral vector constructs described above.

These and other aspects of the present invention will become evident upon reference to the following detailed description and attached drawings. In addition, various references are set forth below which describe in more detail certain procedures or compositions (*e.g*., plasmids, etc.).

### Brief Description of the Drawings

Figure 1 is a schematic illustration of pKS2+Eco57I-LTR(+).
Figure 2 is a schematic illustration of pKS2+Eco57I-LTR(-).
Figure 3 is a schematic illustration of pKS2+LTR-EcoRI.
Figure 4 is a schematic illustration of pR1.
Figure 5 is a schematic illustration of pR2.
Figure 6 is a schematic illustration of pKT1.
Figure 7 is a schematic illustration of pR1-HIVenv.
Figure 8 is a schematic illustration of pR2-HIVenv.
Figure 9 is a representative "prewobble" sequence for a MoMLV *gag*/*pol* (*see also* SEQ I.D. Nos. 11 and 12).
Figure 10 is a representative "wobble" sequence for a MoMLV *gag*/*pol* (*see also* SEQ. I.D. Nos. 9 and 10).
Figure 11 is a schematic illustration of pHCMV-PA.
Figure 12 is a schematic illustration of pCMV *gaglpol.*

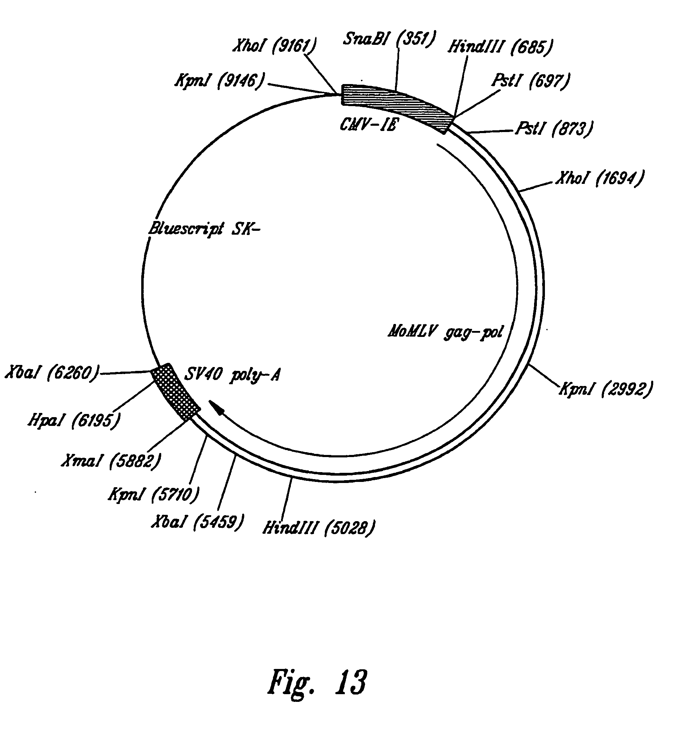

Figure 13 is a schematic illustration of pCMVgpSma.
Figure 14 is a schematic illustration of pCMVgp-X.
Figure 15 is a schematic illustration of pCMV env-X.
Figure 16 is a schematic illustration of pRgpNeo.
Figures 17A, B and C comprise a table which sets forth a variety of retroviruses which may be utilized to construct the retroviral vector constructs, *gag*/*pol* expression cassettes and *env* expression cassettes useful in the present invention.
Figure 18 is a schematic illustration of pCMV Envam-Eag-X-less.
Figure 19A is a diagrammatic illustration of a "wobble" *-gag* construct.
Figure 19B is a diagrammatic illustration of a "normal" *-gag* construct.

### Detailed Description of the Invention

Prior to setting forth the invention, it may be helpful to an understanding thereof to first set forth definitions of certain terms that will be used hereinafter.

"Retroviral vector construct" refers to an assembly which is, within preferred embodiments of the invention, capable of directing the expression of a sequence(s) or gene(s) of interest. Briefly, the retroviral vector construct must include a 5' LTR, a tRNA binding site, a packaging signal, one or more heterologus sequences, an origin of second strand DNA synthesis and a 3' LTR. A wide variety of heterologous sequences may be included within the vector construct, including for example, sequences which encode a protein (*e.g*., cytotoxic protein, disease-associated antigen, immune accessory molecule, or replacement gene), or which are useful as a molecule itself (e.g., as a ribozyme or antisense sequence). Alternatively, the heterologous sequence may merely be a "stuffer" or "filler" sequence, which is of a size sufficient to allow production of viral particles containing the RNA genome. Preferably, the heterologous sequence is at least 1, 2, 3, 4, 5, 6, 7 or 8 kB in length.

The retroviral vector construct may also include transcriptional promoter/enhancer or locus defining element(s), or other elements which control gene expression by means such as alternate splicing, nuclear RNA export, post-translational modification of messenger, or post-transcriptional modification of protein. Optionally, the retroviral vector construct may also include selectable markers such as Neo, TK, hygromycin, phleomycin, histidinol, or DHFR, as well as one or more specific restriction sites and a translation termination sequence.

"Expression cassette" refers to an assembly which is capable of directing the expression of the sequence(s) or gene(s) of interest. The expression cassette must include a promoter which, when transcribed, is operably linked to the sequence(s) or gene(s) of interest, as well as a polyadenylation sequence. Within preferred embodiments of the invention, both the promoter and the polyadenylation sequence are from a source which is heterologous to the helper elements (*i.e., gag*/*pol* and *env*). Expression cassettes of the present invention may be utilized to express a *gag*/*pol* gene or an *env* gene. In addition, the expression cassettes may also be utilized to express one or more heterologous sequences either from a *gag*/*pol* and/or *env* expression cassette, or from a entirely different expression cassette.

Within preferred embodiments useful in the invention, the expression cassettes described herein may be contained within a plasmid construct. In addition to the components of the expression cassette, the plasmid construct may also include a bacterial origin of replication, one or more selectable markers, a signal which allows the plasmid construct to exist as single-stranded DNA (*e.g*., a M13 origin of replication), a multiple cloning site, and a "mammalian" origin of replication (*e.g*., a SV40 or adenovirus origin of replication).

### PREPARATION OF RETROVIRAL VECTOR CONSTRUCTS, GAG/POL EXPRESSION CASSETTES AND ENV EXPRESSION CASSETTES

As noted above, the present invention provides compositions and methods for constructing packaging cells which preclude the formation of replication competent virus by homologous recombination. The following sections describe the preparation of retroviral vector constructs, *gag*/*pol* expression cassettes, and *env* expression cassettes.

### 1. Construction of retroviral vector constructs

Retroviral vector constructs are described comprising a 5' LTR, a tRNA binding site, a packaging signal, one or more heterologous sequences, an origin of second strand DNA synthesis and a 3' LTR, wherein the vector construct lacks *gag*/*pol* or *env* coding sequences. Briefly, Long Terminal Repeats ("LTRs") are subdivided into three elements, designated U5, R and U3. These elements contain a variety of signals which are responsible for the biological activity of a retrovirus, including for example, promoter and enhancer elements which are located within U3. LTR's may be readily identified in the provirus due to their precise duplication at either end of the genome.

The tRNA binding site and origin of second strand DNA synthesis are also important for a retrovirus to be biologically active, and may be readily identified by one of skill in the art. For example, tRNA binds to a retroviral tRNA binding site by Watson-Crick base pairing, and is carried with the retrovirus genome into a viral particle. The tRNA is then utilized as a primer for DNA synthesis by reverse transcriptase. The tRNA binding site may be readily identified based upon its location just downstream from the 5' LTR. Similarly, the origin of second strand DNA synthesis is, as its name implies, important for the second strand DNA synthesis of a retrovirus. This region, which is also referred to as the poly-purine tract, is located just upstream of the 3' LTR.

In addition to 5' and 3' LTRs, a tRNA binding site, and an origin of second strand DNA synthesis, retroviral vector constructs of the present invention also comprise a packaging signal, as well as one or more heterologous sequences, each of which is discussed in more detail below.

Retroviral vector constructs used in the present invention may be readily construed from a wide variety of retroviruses, including for example, B, C, and D type retroviruses as well as spumaviruses and lentiviruses (*see* RNA Tumor Viruses, Second Edition, Cold Spring Harbor Laboratory, 1985). Briefly, viruses are often classified according to their morphology as seen under electron microscopy. Type "B" retroviruses appear to have an eccentric core, while type "C" retroviruses have a central core. Type "D" retroviruses have a morphology intermediate between type B and type C retroviruses. Representative examples of suitable retroviruses include those set forth below in Figures 17A, B and C (*see* RNA Tumor Viruses at pages 2-7), as well as a variety of xenotropic retroviruses (*e.g*., NZB-X1, NZB-X2 and NZB₉₋₁ (*see* O'Neill et al., J. Vir. 53:100-106, 1985)) and polytropic retroviruses (*e.g.,* MCF and MCF-MLV (*see* Kelly et al., J. Vir. 45(1):291-298, 1983)). Such retroviruses may be readily obtained from depositories or collections such as the American Type Culture Collection ("ATCC"; Rockville, Maryland), or isolated from known sources using commonly available techniques.

Particularly preferred retroviruses for the preparation or construction of retroviral vector constructs useful in the present invention include retroviruses selected from the group consisting of Avian Leukosis Virus, Bovine Leukemia Virus, Murine Leukemia Virus, Mink-Cell Focus-Inducing Virus, Murine Sarcoma Virus, Reticuloendotheliosis virus, Gibbon Ape Leukemia Virus, Mason Pfizer Monkey Virus, and Rous Sarcoma Virus. Particularly preferred Murine Leukemia Viruses include 4070A and 1504A (Hartley and Rowe, J. Virol. 19:19-25, 1976), Abelson (ATCC No. VR-999), Friend (ATCC No. VR-245), Graffi, Gross (ATCC No. VR-590), Kirsten, Harvey Sarcoma Virus and Rauscher (ATCC No. VR-998), and Moloney Murine Leukemia Virus (ATCC No. VR-190). Particularly preferred Rous Sarcoma Viruses include Bratislava, Bryan high titer (*e.g*., ATCC Nos. VR-334, VR-657, VR-726, VR-659, and VR-728), Bryan standard, Carr-Zilber, Engelbreth-Holm, Harris, Prague (*e.g.,* ATCC Nos. VR-772, and 45033), and Schmidt-Ruppin (*e.g*. ATCC Nos. VR-724, VR-725, VR-354).

Any of the above retroviruses may be readily utilized in order to assemble or construct retroviral vector constructs useful in the invention, or packaging cells or producer cells of the present invention given the disclosure provided herein, and standard recombinant techniques (*e.g*., Sambrook et al, Molecular Cloning: A Laboratory Mamial, 2d ed., Cold Spring Harbor Laboratory Press, 1989; Kunkle, PNAS 82:488, 1985). Further, within certain embodiments of the invention, portions of the retroviral vector construct may be derived from different retroviruses. For example, within one embodiment of the invention, retrovector LTRs may be derived from a Murine Sarcoma Virus, a tRNA binding site from a Rous Sarcoma Virus, a packaging signal from a Murine Leukemia Virus, and an origin of second strand synthesis from an Avian Leukosis Virus. Similarly, portions of a packaging cell line may be derived from different viruses (*e.g.,* a *gag*/*pol* expression cassette may be constructed from a Moloney Murine Leukemia Virus, and an *env* expression cassette from a Mason Pfizer Monkey virus).

As noted above, within various aspects of the present invention, retroviral vector constructs are used which have packaging signals, and which lack both *gag*/*pol* and *env* coding sequences. As utilized within the context of the present invention, a packaging signal should be understood to refer to that sequence of nucleotides which is not required for synthesis, processing or translation of viral RNA or assembly of virions, but which is required in *cis* for encapsidation of genomic RNA (*see* Mann et al., Cell 33:153-159, 1983; RNA Tumor Viruses, Second Edition, *supra*). Further, as utilized herein, the phrase "lacks *gag*/*pol* or *env* coding sequences" should be understood to refer to retrovectors which contain less than 20, preferably less than 15, more preferably less than 10, and most preferably less than 8 consecutive nucleotides which are found in *gag*/*pol* or *env* genes, and in particular, within *gag*/*pol* or *env* expression cassettes that are used to construct packaging cell lines for the retroviral vector construct. Representative examples of such retroviral vector constructs are set forth in more detail below and in Example 1.

As an illustration, within one embodiment useful in the invention construction of retroviral vector constructs which lack *gag*/*pol* or *env* sequences may be accomplished by preparing retroviral vector constructs which lack an extended packaging signal. As utilized herein, the phrase "extended packaging signal" refers to a sequence of nucleotides beyond the minimum core sequence which is required for packaging, that allows increased viral titer due to enhanced packaging. As an example, for the Murine Leukemia Virus MoMLV, the minimum core packaging signal is encoded by the sequence (counting from the 5' LTR cap site) from approximately nucleotide 144 of SEQ. I.D. No. 1, up through the *Pst* 1 site (nucleotide 567 of SEQ. I.D. No.1). The extended packaging signal of MoMLV includes the sequence beyond nucleotide 567 up through the start of the *gag*/*pol* gene (nucleotide 621), and beyond nucleotide 1040. Thus, within this embodiment retroviral vector constructs which lack extended packaging signal may be constructed from the MoMLV by deleting or truncating the packaging signal downstream of nucleotide 567.

Within other embodiments of the invention, retroviral vector constructs are useful wherein the packaging signal that extends into, or overlaps with, retroviral *gag*/*pol* sequence is deleted or truncated. For example, in the representative case of MoMLV, the packaging signal is deleted or truncated downstream of the start of the *gag*/*pol* gene (nucleotide 621 of SEQ ID NO: 1). Within preferred embodiments of the invention, the packaging signal is terminated at nucleotide 570, 575, 580, 585, 590, 595, 600, 610, 615 or 617 of SEQ ID NO: 1.

Within other aspects of the invention, retroviral vector constructs are useful which include a packaging signal that extends beyond the start of the *gag*/*pol* gene (*e.g*., for MoMLV, beyond nucleotide 621 of SEQ ID NO: 1). When such retroviral vector constructs are utilized, it is preferable to utilize packaging cell lines for the production of recombinant viral particles wherein the 5' terminal end of the *gag*/*pol* gene in a *gag*/*pol* expression cassette has been modified to contain codons which are degenerate for *gag.* Such *gag*/*pol* expression cassettes are described in more detail below in section 2, and in Example 3.

Within other aspects of the present invention, retroviral vector constructs are useful comprising a 5' LTR, a tRNA binding site, a packaging signal, an origin of second strand DNA synthesis and a 3' LTR, wherein the retrovector plasmid construct does not contain a retroviral nucleic acid sequence upstream of the 5' LTR. As utilized within the context of the present invention, the phrase "does not contain a retroviral nucleic acid sequence upstream of the 5' LTR" should be understood to mean that the retrovector plasmid construct contains less than 20, preferably less than 15, more preferably less than 10, and most preferably less than 8 consecutive nucleotides which are found in a retrovirus, and more specifically, in a retrovirus which is homologous to the retroviral vector construct, upstream of and/or contiguous with the 5' LTR. Within preferred embodiments, the retrovector plasmid constructs do not contain an *env* coding sequence (as discussed below) upstream of the 5' LTR. A particularly preferred embodiment of such retrovector plasmid constructs is set forth in more detail below in Example 1.

Within a further aspect of the present invention, retrovector plasmid constructs are used comprising a 5' LTR, a tRNA binding site, a packaging signal, an origin of second strand DNA synthesis and a 3' LTR, wherein the retrovector plasmid construct does not contain a retroviral packaging signal sequence downstream of the 3' LTR. As utilized herein, the term "packaging signal sequence" should be understood to mean a sequence sufficient to allow packaging of the RNA genome. A representative example of such a retroviral vector construct is set forth in more detail below in Example 1.

### 2. Construction of gag/pol expression cassettes

A variety of *gag*/*pol* expression cassettes, in combination with the retroviral vector constructs and *env* expression cassettes of the present invention, may enable the construction of packaging cell lines and producer cell lines which preclude the formation of replication competent virus. Briefly, retroviral *gaglpol* genes contain a *gag* region which encodes a variety of structural proteins that make up the core matrix and nucleocapsid, and a *pol* region which contains genes which encode (1) a protease for the processing of *gaglpol* and *env* proteins, (2) a reverse transcriptase polymerase, (3) an RNase H, and (4) an integrase, which is necessary for integration of the retroviral provector into the host genome. Although retroviral *gag*/*pol* genes may be utilized to construct *gag*/*pol* expression cassettes, a variety of other non-retroviral (and non-viral) genes may also be utilized to construct the *gag*/*pol* expression cassette. For example, a gene which encodes retroviral RNase H may be replaced with genes which encode bacterial (*e.g., E. coli* or *Thermus thermophilus*) RNase H. Similarly, a retroviral integrase gene may be replaced by other genes with similar function (*e.g*., yeast retrotransposon TY3 integrase).

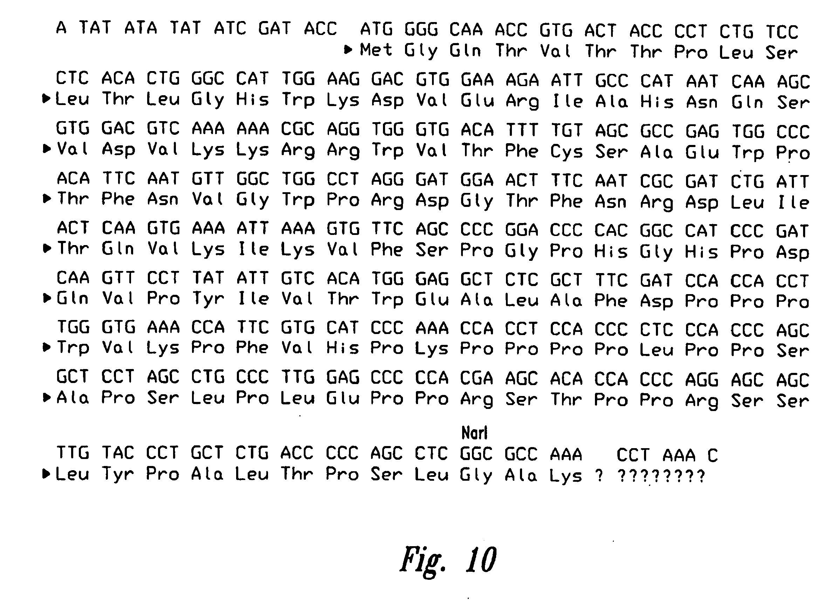

*gag*/*pol* expression cassettes may comprise a promoter operably linked to a *gag*/*pol* gene, and a polyadenylation sequence, wherein the *gag*/*pol* gene has been modified to contain codons which are degenerate for gag. Briefly, as noted above, in wild-type retrovirus the extended packaging signal of the retrovirus overlaps with sequences which encode gag and pol. Thus, in order to eliminate the potential of crossover between the retroviral vector construct and the *gaglpol* expression cassette, as well as to eliminate the possiblity of co-encapsidation of the *gag*/*pol* expression cassette and replication competent virus or retroviral vector constructs, sequences of overlap should be eliminated. Elimination of such overlap may be accomplished by modifying the *gag*/*pol* gene (and more specifically, regions which overlap with the retroviral vector construct, such as the extended packaging signal) to contain codons that are degenerate (*i.e*., that "wobble") for gag. In particular, within preferred embodiments of the invention codons are selected which encode biologically active gag/pol protein (*i.e*., capable of producing a competent retroviral particle, in combination with an *env* expressing element, and a RNA genome), and which lack any packaging signal sequence, including in particular, extended packaging signal sequence. As utilized herein, the phrase "lacks any retroviral packaging signal sequence" should be understood to mean that the *gaglpol* expression cassette contains less than 20, preferably less than 15, more preferably less than 10, and most preferably less than 8 consecutive nucleotides which are identical to a sequence found in a retroviral packaging signal (*e.g.,* in the case of MoMLV, extending up and through the *Xho* I site at approximately nucleotide number 1561). A particularly preferred example of such modified codons which are degenerate for *gag* is shown in Figure 10, and in Example 3. In particular, within other embodiments, at least 25, 50, 75, 100, 125 or 135 *gag* codons are modified or "wobbled" from the native *gag* sequence within the *gag*/*pol* expression cassettes of the present invention.

In addition to eliminating overlap between the retroviral vector construct and the *gag*/*pol* gene, it is also preferable to eliminate any potential overlap between the *gag*/*pol* gene and the *env* gene in order to prohibit the possibility of homologous recombination. This may be accomplished in at least two principal ways: (1) by deleting a portion of the *gag*/*pol* gene which encodes the integrase protein, and in particular, that portion of the gene which encodes the integrase protein which overlaps with the *env* coding sequence, or (2) by selecting codons which are degenerate for integrase and/or env.

Thus, *gag*/*pol* expression cassettes may be used comprising a promoter operably linked to a *gag*/*pol* gene, and a polyadenylation sequence or signal, wherein a 3' terminal end of the gene has been deleted without effecting the biological activity of the integrase. (The biological activity of integrase may be readily determined by detection of an integration event, either by DNA analysis or by expression of a transduced gene; *see* Roth et al., J. Vir. 65(4):2141-2I45, 1991.) As an example, in the Murine Leukemia Virus MoMLV (SEQ ID. NO. 1), the *gaglpol* gene is encoded by nucleotides 621 through 5834. Within this sequence, the protein integrase is encoded by nucleotides 4610 through nucleotide 5834. A portion of the *gaglpol* sequence which encodes integrase also encodes env (which begins at nucleotide 5776). Thus, within one embodiment of the invention, the 3' terminal end of the *gaglpol* gene is deleted or truncated in order to prevent crossover with the *env* gene, without effecting the biological activity of the integrase. Within other preferred embodiments, the *gag*/*pol* gene is deleted at any nucleotide downstream (3') from the beginning of the integrase coding sequence, and preferably prior to the start of the *env* gene sequence. Within one embodiment, the sequence encoding gag/pol is a MoMLV sequence, and the *gag*/*pol* gene is deleted at any nucleotide between nucleotides 4610 and 5576 (of SEQ. I.D. No. 1), including for example, at nucleotides 5775, 5770, 5765, 5760, 5755, 5750.

Within other embodiments of the invention, the *gaglpol* expression cassette contains sequences encoding gag/pol (and including integrase), while lacking any sequence found in an *env* gene. The phrase "lacking any sequence found in an *env* gene" should be understood to mean that the *gag*/*pol* expression cassette does not contain at least 20, preferably at least 15, more preferably at least 10, and most preferably less than 8 consecutive nucleotides which are identical to an *env* sequence, and preferably which are found in an *env* expression cassette which will be utilized along with the *gag*/*pol* expression cassette to form a packaging cell. Such expression cassettes may be readily prepared by selecting codons which are degenerate for integrase, and which do not encode biologically active env. (*See* Morgenstern and Land, Nuc. Acids Res. 18:3587-3596, 1990.)

Within other embodiments of the invention, the *gag*/*pol* expression cassette contains a heterologous promoter, and/or heterologous polyadenylation sequence. As utilized herein, "heterologous" promoters or polyadenylation sequences refers to promoters or polyadenylation sequences which are from a different source from which the *gaglpol* gene (and preferably the *env* gene and retroviral vector construct) is derived from. Representative examples of suitable promoters include the Cytomegalovirus Immediate Early ("CMV IE") promoter, the Herpes Simplex Virus Thymidine Kinase ("HSVTK") promoter, the Rous Sarcoma Virus ("RSV") promoter, the Adenovirus major-late promoter and the SV 40 promoter. Representative examples of suitable polyadenylation signals include the SV 40 late polyadenylation signal and the SV40 early polyadenylation signal.

Within preferred aspects of the present invention, *gag*/*pol* expression cassettes such as those described above will not co-encapsidate along with a replication competent virus. One representative method for determination of co-encapsidation is set forth below in Example 8.

### 3. Construction of env expression cassettes

*env* expression cassettes may be provided which, in combination with the *gag*/*pol* expression cassettes and retroviral vector constructs described above, preclude formation of replication competent virus by homologous recombination, as well as to confer a particular specificity of the resultant vector particle (*e.g*., amphotropic, ecotropic, xenotropic or polytropic; see Figure 17, as well as the discussion above). Briefly, in a wild-type retrovirus the *env* gene encodes two principal proteins, the surface glycoprotein "SU" and the transmembrane protein "TM", which are translated as a polyprotein, and subsequently separated by proteolytic cleavage. Representative examples of the SU and TM proteins are the gp120 protein and gp41 protein in HIV, and the gp70 protein and p15e protein in MoMLV. In some retroviruses, a third protein designated the "R" peptide" of undetermined function, is also expressed from the *env* gene and separated from the polyprotein by proteolytic cleavage. In the Murine Leukemia Virus MoMLV, the R peptide is designated "p2".

A wide variety of *env* expression cassettes may be constructed given the disclosure provided herein, and utilized within the present invention to preclude homologous recombination. *env* expression cassettes may be provided comprising a promoter operably linked to an *env* gene, wherein no more than 6, 8, 10, 15, or 20 consecutive retroviral nucleotides are included upstream (5') of and/or contiguous with said *env* gene. *env* expression cassettes may be provided comprising a promoter operably linked to an *env* gene, wherein the *env* expression cassette does not contain a consecutive sequence of greater than 20, preferably less than 15, more preferably less than 10, and most preferably less than 8 or 6 consecutive nucleotides which are found in a *gag*/*pol* gene, and in particular, in a *gag*/*pol* expression cassette that will be utilized along with the *env* expression cassette to create a packaging cell line.

*env* expression cassettes may be provided comprising a promoter operably linked to an *env* gene, and a polyadenylation sequence, wherein a 3' terminal end of the *env* gene has been deleted without effecting the biological activity of env. As utilized herein, the phrase "biological activity of env" refers to the ability of envelop protein to be expressed on the surface of a virus or vector particle, and to allow for a successful infection of a host cell. One practical method for assessing biological activity is to transiently transfect the *env* expression cassette into a cell containing a previously determined functional *gag*/*pol* expression cassette, and a retroviral vector construct which expresses a selectable marker. A biologically functional *env* expression cassette will allow vector particles produced in that transfected cell, to transmit the selectable marker to a naive sensitive cell such that it becomes resistant to the marker drug selection. Within a preferred embodiment of the invention, the 3' terminal end of the *env* gene is deleted or truncated such that a complete R peptide is not produced by the expression cassette. In the representative example of MoMLV, sequence encoding the R peptide (which begins at nucleotide 7734) is deleted, truncated, or, for example, terminated by insertion of a stop codon at nucleotide 7740, 7745, 7747, 7750, 7755, 7760, 7765, 7770, 7775, 7780, or any nucleotide in between.

*env* expression cassettes may be provided which contain a heterologous promoter, and/or heterologous polyadenylation sequence. As utilized herein, "heterologous" promoters or polyadenylation sequences refers to promoters or polyadenylation sequences which are from a different source from which the *gag*/*pol* gene (and preferably the *env* gene and retroviral vector construct) is derived from. Representative examples of suitable promoters include the CMV IE promoter, the HSVTK promoter, the RSV promoter, the Adenovirus major-late promoter and the SV 40 promoters. Representative examples of suitable polyadenylation signals include the SV 40 late polyadenylation signal and the SV40 early polyadenylation signal.

### HETEROLOGOUS SEQUENCES

As noted above, the retroviral vector constructs, *gag*/*pol* expression cassettes, and *env* expression cassettes described herein may contain (and express) one or more heterologous sequences. A wide variety of heterologous sequences may be utilized within the context of the present invention, including for example, cytotoxic genes, antisense sequences, sequences which encode gene products that activate a compound with little or no cytotoxicity (*i.e*., a "prodrug") into a toxic product, sequences which encode immunogenic portions of disease-associated antigens and sequences which encode immune accessory molecules. Representative examples of cytotoxic genes include the genes which encode proteins such as ricin (Lamb et al., Eur. J. Biochem. 148:265-270, 1985), abrin (Wood et al., Eur. J. Biochem. 198:723-732, 1991; Evensen, et al., J. of Biol. Chem. 266:6848-6852, 1991: Collins et al., J. of biol Chem. 265:8665-8669, 1990; Chen et al., Fed of Eur. Biochem Soc. 309:115-118, 1992), diphtheria toxin (Tweten et al., J. Biol. Chem. 260:10392-10394, 1985), cholera toxin (Mekalanos et al., Nature 306:551-557, 1983; Sanchez & Holmgren, PNAS 86:481-485, 1989), gelonin (Stirpe et al., J. Biol. Chem. 255:6947-6953, 1980), pokeweed (Irvin, Pharmac. Ther. 21:371-387, 1983), antiviral protein (Barbieri et al., Biochem. J. 203:55-59, 1982; Irvin et al., Arch. Biochem. & Biophys. 200:418-425, 1980; Irvin, Arch. Biochem. & Biophys 169:522-528, 1975), tritin, Shigella toxin (Calderwood et al., PNAS 84:4364-4368, 1987; Jackson et al., Microb. Path. 2:147-153, 1987), and Pseudomonas exotoxin A (Carroll and Collier, J. Biol. Chem. 262:8707-8711, 1987).

Antisense RNA may be utilized as a cytotoxic gene in order to induce a potent Class I restricted response. Briefly, in addition to binding RNA and thereby preventing translation of a specific mRNA, high levels of specific antisense sequences may be utilized to induce the increased expression of interferons (including gamma-interferon), due to the formation of large quantities of double-stranded RNA. The increased expression of gamma interferon, in turn, boosts the expression of MHC Class I antigens. Preferred antisense sequences for use in this regard include actin RNA, myosin RNA, and histone RNA. Antisense RNA which forms a mismatch with actin RNA is particularly preferred.

Antisense sequences are described which inhibit, for example, tumour cell growth, viral replication, or a genetic disease by preventing the cellular synthesis of critical proteins needed for cell growth. Examples of such antisense sequences include antisense thymidine kinase, antisense dihydrofolate reductase (Maher and Dolnick, Arch. Biochem. & Biophys. 253:214-220, 1987; Bzik et al., PNAS 84:8360-8364, 1987), antisense HER2 (Coussens et al., Science 230:1132-1139, 1985), antisense ABL (Fainstein, et al., Oncogene 4:1477-1481, 1989), antisense Myc (Stanton et al., Nature 310:423-425, 1984) and antisense *ras,* as well as antisense sequences which block any of the enzymes in the nucleotide biosynthetic pathway.

Retroviral vector constructs, *gag*/*pol* expression cassettes and *env* expression cassettes are described which direct the expression of a gene product that activates a compound with little or no cytotoxicity (*i.e*., a "prodrug") into a toxic product. Representative examples of such gene products include varicella zoster virus thymidine kinase (VZVTK), herpes simplex virus thymidine kinase (HSVTK) (Field et al., J. Gen. Virol. 49:115-124, 1980; Munir et al., Protein Engineering 7(1):83-89, 1994; Black and Loeb, Biochem 32(43):11618-11626, 1993), and *E. coli*. guanine phosphoribosyl transferase (*see* U.S. Patent Application Serial No. 08/155,944, entitled "Compositions and Methods for Utilizing Conditionally Lethal Genes," filed November 18, 1993; *see also* WO 93/10218 entitled "Vectors Including Foreign Genes and Negative Selection Markers", WO 93/01281 entitled "Cytosine Deaminase Negative Selection System for Gene Transfer Techniques and Therapies", WO 93/08843 entitled "Trapped Cells and Use Thereof as a Drug", WO 93/08844 entitled "Transformant Cells for the Prophylaxis or Treatment of Diseases Caused by Viruses, Particularly Pathogenic Retroviruses", and WO 90/07936 entitled "Recombinant Therapies for Infection and Hyperproliferative Disorders.") Retroviral vector constructs are described which direct the expression of a gene product that activates a compound with little or no cytotoxicity into a toxic product in the presence of a pathogenic agent, thereby affecting localized therapy to the pathogenic agent *(see* WO 94/13304).

Retroviral vector constructs are described which direct the expression of a HSVTK gene downstream, and under the transcriptional control of an HIV promoter (which is known to be transcriptionally silent except when activated by HIV tat protein). Briefly, expression of the tat gene product in human cells infected with SHIV and carrying the vector construct causes increased production of HSVTK. The cells (either *in vitro* or *in vivo*) are then exposed to a drug such as ganciclovir, acyclovir or its analogues (FIAU, FIAC, DHPG). Such drugs are known to be phosphorylated by HSVTIC (but not by cellular thymidine kinase) to their corresponding active nucleotide triphosphate forms. Acyclovir and FIAU triphosphates inhibit cellular polymerases in general, leading to the specific destruction of cells expressing HSVTK in transgenic mice (*see* Borrelli et al., Proc. Natl. Acad. Sci. USA 85:7572, 1988). Those cells containing the recombinant vector and expressing HIV tat protein are selectively killed by the presence of a specific dose of these drugs.

Retroviral vector constructs, *gag*/*pol* expression cassettes and *env* expression cassettes useful in the present invention may also direct the expression of one or more sequences which encode immunogenic portions of disease-associated antigens. As utilized within the context of the present invention, antigens are deemed to be "disease-associated" if they are either associated with rendering a cell (or organism) diseased, or are associated with the disease-state in general but are not required or essential for rendering the cell diseased. In addition, antigens are considered to be "immunogenic" if they are capable, under appropriate conditions, of causing an immune response (either cell-mediated or humoral). Immunogenic "portions" may be of variable size, but are preferably at least 9 amino acids long, and may include the entire antigen.

A wide variety of "disease-associated" antigens are contemplated as being useful in the present invention, including for example immunogenic, non-tumorigenic forms of altered cellular components which are normally associated with tumor cells (*see* WO 93/10814). Representative examples of altered cellular components which are normally associated with tumor cells include ras* (wherein "*" is understood to refer to antigens which have been altered to be non-tumorigenic), p53*, Rb*, altered protein encoded by Wilms' tumor gene, ubiquitin*, mucin, protein encoded by the DCC. APC, and MCC genes, as well as receptors or receptor-like structures such as neu, thyroid hormone receptor, Platelet Derived Growth Factor ("PDGF") receptor, insulin receptor, Epidermal Growth Factor ("EGF") receptor, and the Colony Stimulating Factor ("CSF") receptor.

"Disease-associated" antigens should also be understood to include all or portions of various eukaryotic, prokaryotic or viral pathogens. Representative examples of viral pathogens include the Hepatitis B Virus ("HBV") and Hepatitis C Virus ("HCV"; *see* WO 93/15207), Human Papiloma Virus ("HPV"; *see* WO 92/05248; WO 90/10459; EPO 133,123), Epstein-Barr Virus ("EBV"; *see* EPO 173,254; JP 1,128,788; and U.S. Patent Nos. 4,939,088 and 5,173.414), Feline Leukemia Virus ("FeLV"; *see* WO 93/09070; EPO 377,842; WO 90/08832; WO 93/09238), Feline Immunodeficiency Virus ("FIV"; U.S. Patent No. 5,037,753; WO 92/15684; WO 90/13573; and JP 4,126,085), HTLV I and II, and Human Immunodeficiency Virus ("FHV"; *see* WO 93/02805).

The retroviral vector constructs, *gag*/*pol* expression cassettes and *env* expression cassettes described above may also direct the expression of one or more immune accessory molecules. As utilized herein, the phrase "immune accessory molecules" refers to molecules which can either increase or decrease the recognition, presentation or activation of an immune response (either cell-mediated or humoral). Representative examples of immune accessory molecules include α interferon, β interferon, IL-1, IL-2, IL-3, IL-4, IL-5. IL-6, IL-7 (U.S. Patent No. 4,965,195), IL-8, IL-9, IL-10, IL-11, IL-12 (Wolf et al., J. Immun. 46:3074, 1991; Gubler et al., PNAS 88:4143, 1991; WO 90/05147; EPO 433,827), IL-13 (WO 94/04680), IL-14, IL-15, GM-CSF, M-CSF-1, G-CSF, CD3 (Krissanen et al., Immunogenetics 26:258-266, 1987), CD8, ICAM-1 (Simmons et al., Nature 331:624-627, 1988), ICAM-2 (Singer, Science 255: 1671, 1992), β-microglobulin (Parnes et al., PNAS 78:2253-2257, 1981), LFA-1 (Altmann et al., Nature 338: 521, 1989), LFA3 (Wallner et al., J. Exp. Med 166(4):923-932, 1987), HLA Class I, HLA Class II molecules, B7 (Freeman et al., J. Immun. 143:2714, 1989), and B7-2. The heterologous gene may encode gamma-interferon.

The retroviral vector constructs described herein may direct the expression of more than one heterologous sequence. Such multiple sequences may be controlled either by a single promoter, or preferably, by additional secondary promoters (*e.g*., Internal Ribosome Binding Sites or "IRBS"). Retroviral vector constructs direct the expression of heterologous sequences which act synergistically. For example, retroviral vector constructs are described which direct the expression of a molecule such as IL-15, IL-12, IL-2, gamma interferon, or other molecule which acts to increase cell-mediated presentation in the T_{H}1 pathway, along with an immunogenic portion of a disease-associated antigen. Immune presentation and processing of the disease-associated antigen will be increased due to the presence of the immune accessory molecule.

Retroviral vector constructs are described which direct the expression of one or more heterologous sequences which encode "replacement" genes. As utilized herein, it should be understood that the term "replacement genes" refers to a nucleic acid molecule which encodes a therapeutic protein that is capable of preventing, inhibiting, stabilizing or reversing an inherited or noninherited genetic defect. Representative examples of such genetic defects include disorders in metabolism, immune regulation, hormonal regulation, and enzymatic or membrane associated structural function. Representative examples of diseases caused by such defects include Cystic Fibrosis ("CF"; *see* Dorin et al., Nature 326:614, ), Parkinson's Disease, Adenosine Deaminase deficiency ("ADA"; Hahma et al., J. Bact. 173:3663-3672, 1991), β-globin disorders, Hemophilia A & B (Factor VIII-deficiencies; *see* Wood et al., Nature 312:330, 1984), Gaucher disease, diabetes, forms of gouty arthritis and Lesch-Nylan disease (due to "HPRT" deficiencies; *see* Jolly et al., PNAS 80:477-481, 1983) and Familial Hypercholesterolemia (LDL Receptor mutations; *see* Yamamoto et al., Cell 39:27-38, 1984).

Sequences which encode the above-described heterologous genes may be readily obtained from a variety of sources. For example, plasmids which contain sequences that encode immune accessory molecules may be obtained from a depository such as the American Type Culture Collection (ATCC, Rockville, Maryland), or from commercial sources such as British Bio-Technology Limited (Cowley, Oxford England). Representative sources sequences which encode the above-noted immune accessory molecules include BBG 12 (containing the GM-CSF gene coding for the mature protein of 127 amino acids), BBG 6 (which contains sequences encoding gamma interferon), ATCC No. 39656 (which contains sequences encoding TNF), ATCC No. 20663 (which contains sequences encoding alpha interferon), ATCC Nos. 31902, 31902 and 39517 (which contains sequences encoding beta interferon), ATCC No 67024 (which contains a sequence which encodes Interleukin-1). ATCC Nos. 39405, 39452, 39516, 39626 and 39673 (which contains sequences encoding Interleukin-2), ATCC Nos. 59399, 59398, and 67326 (which contain sequences encoding Interleukin-3), ATCC No. 57592 (which contains sequences encoding Interleukin-4), ATCC Nos. 59394 and 59395 (which contain sequences encoding Interleukin-5), and ATCC No. 67153 (which contains sequences encoding Interleukin-6). It will be evident to one of skill in the art that one may utilize either the entire sequence of the protein, or an appropriate portion thereof which encodes the biologically active portion of the protein.

Alternatively, known cDNA sequences which encode cytotoxic genes or other heterologous genes may be obtained from cells which express or contain such sequences. Briefly, mRNA from a cell which expresses the gene of interest is reverse transcribed with reverse transcriptase using oligo dT or random primers may be used. The single stranded cDNA may then be amplified by PCR (*see* U.S. Patent Nos. 4,683,202, 4,683,195 and 4,800,159. *See also* PCR Technology: Principles and Applications for DNA Amplification, Erlich (ed.), Stockton Press, 1989) utilizing oligonucleotide primers complementary to sequences on either side of desired sequences. In particular, a double stranded DNA is denatured by heating in the presence of heat stable Taq polymerase, sequence specific DNA primers, ATP, CTP, GTP and TTP. Double-stranded DNA is produced when synthesis is complete. This cycle may be repeated many times, resulting in a factorial amplification of the desired DNA.

Sequences which encode the above-described genes may also be synthesized, for example, on an Applied Biosystems Inc. DNA synthesizer (*e.g.*, ABI DNA synthesizer model 392 (Foster City, California)).

### PREPARATION OF RETROVIRAL PACKAGING CELL LINES, AND GENERATION OF RECOMBINANT VIRAL PARTICLE

As noted above, the *gag*/pol expression cassettes and *env* expression cassettes useful in the present invention may be used to generate transduction competent retroviral vector particles by introducing them into an appropriate parent cell line in order to create a packaging cell line, followed by introduction of, a retroviral vector construct, in order to create a producer cell line (*see* WO 92/05266). Such packaging cell lines, upon introduction of an N2-type vector construct (Armentano et al., J. of Vir. 61(5):1647-1650, 1987) produce a titer of greater than 10⁵ cfu/ml, and preferably greater than 10-fold, 20-fold, 50-fold, or 100-fold higher titer than similar transduced PA317 cells (Miller and Buttimore, Mol. and Cell. Biol. 6(8):2895-2902, 1986).

Within one aspect of the present invention, methods for creating packaging cell lines are provided, comprising the steps of (a) introducing a *gag*/*pol* expression cassette according into an animal cell, (b) selecting a cell containing a *gaglpol* expression cassette which expresses high levels of *gaglpol,* (c) introducing an env expression cassette into the selected cell, and (d) selecting a cell which expresses high levels of *env,* and thereby creating the packaging cell. Within other aspects of the present invention, methods for creating packaging cell lines are provided comprising the steps of (a) introducing an *env* expression cassette into an animal cell (b) selecting a cell which expresses high levels of *env*, (c) introducing a *gaglpot* expression cassette into the selected cell, and (d) selecting a cell containing a *gaglpol* expression cassette which expresses high levels of *gaglpol,* and thereby creating the packaging cell. As utilized herein, it should be understood that "high" levels of gag/pol or env refers to packaging cells which produce at least z times greater gag/pol or env protein than PA317 cells, wherein z is selected from the group consisting of 1, 2, 3, 4, 5, 6, 7, 8, 9 or 10.

A wide variety of animal cells may be utilized to prepare the packaging cells of the present invention, including for example human, macaque, dog, rat and mouse cells. Particularly preferred cell lines for use in the preparation of packaging cell lines of the present invention are those that lack genomic sequences which are homologous to the retroviral vector construct, *gaglpol* expression cassette and *env* expression cassette to be utilized. Methods for determining homology may be readily accomplished by, for example, hybridization analysis (*see* Martin et al., PNAS 78:4892-4896, 1981; *see also* WO 92/05266).

Expression cassettes useful in the present invention may be introduced into cells by numerous techniques, including for example, transfection by various physical methods, such as electroporation, DEAE dextran, lipofection (Felgner et al., Proc. Natl. Acad Sci. USA 84:7413-7417, 1989), direct DNA injection (Acsadi et al., Nature 352:815-818, 1991); microprojectile bombardment (Williams et al., PNAS 88:2726-2730, 1991), liposomes of several types (*see e.g.,* Wang et al., PNAS 84:7851-7855, 1987); CaPO₄ (Dubensky et al., PNAS 81:7529-7533, 1984), DNA ligand (Wu et al, J. of Biol. Chem. 264:16985-16987, 1989), administration of nucleic acids alone (WO 90/11092), or administration of DNA linked to killed adenovirus (Curiel et al., Hum. Gene Ther. 3: 147-154, 1992).

Producer cell lines (also called vector-producing lines or "VCLs") may then be readily prepared by introducing a retroviral vector construct as described above, into a packaging cell line. Within preferred embodiments of the invention, producer cell lines are provided comprising a *gaglpol* expression cassette, an *env* expression cassette, and a retroviral vector construct, wherein the *gaglpol* expression cassette, *env* expression cassette and retroviral vector construct lack a consecutive sequence of greater than 20, preferably 15, more preferably 10, and most preferably 10 or 8 nucleotides in common.

### PHARMACEUTICAL COMPOSITIONS

Pharmaceutical compositions may be provided, comprising a recombinant viral particle as described above, in combination with a pharmaceutically acceptable carrier or diluent. Such pharmaceutical compositions may be prepared either as a liquid solution, or as a solid form (*e.g*., lyophilized) which is suspended in a solution prior to administration. In addition, the composition may be prepared with suitable carriers or diluents for topical administration, injection, or oral, nasal, vaginal, sub-lingual, inhalant or rectal administration.

Pharmaceutically acceptable carriers or diluents are nontoxic to recipients at the dosages and concentrations employed. Representative examples of carriers or diluents for injectable solutions include water, isotonic saline solutions which are preferably buffered at a physiological pH (such as phosphate-buffered saline or Tris-buffered saline), mannitol, dextrose, glycerol, and ethanol, as well as polypeptides or proteins such as human serum albumin. A particularly preferred composition comprises a retroviral vector construct or recombinant viral particle in 10 mg/ml mannitol, 1 mg/ml HSA, 20 mM Tris, pH 7.2, and 150 mM NaCl. In this case, since the recombinant vector represents approximately I mg of material, it may be less than 1% of high molecular weight material, and less than 1/100,000 of the total material (including water). This composition is stable at -70°C for at least six months.

Pharmaceutical compositions may also additionally include factors which stimulate cell division, and hence, uptake and incorporation of a recombinant retroviral vector. Representative examples include Melanocyte Stimulating Hormone (MSH), for melanomas or epidermal growth factor for breast or other epithelial carcinomas.

Particularly preferred methods and compositions for preserving recombinant viruses are described in U.S. applications entitled "Methods for Preserving Recombinant Viruses" (*see* WO 94/11414).

### METHODs OF ADMINTSTRATION

Methods may be provided for inhibiting or destroying pathogenic agents in a warm-blooded animal, comprising administering to a warm-blooded animal a recombinant viral particle as described above, such that the pathogenic agent is inhibited or destroyed. Recombinant viral particles may be administered *in vivo,* or *ex vivo.* Representative routes for *in vivo* administration include intradermally ("i.d."), intracranially ("i.c."), intraperitoneally ("i.p."), intrathecally ("i.t."), intravenously ("i.v."), subcutaneously ("s.c."), intramuscularly ("i.m.") or even directly into a tumor.

Alternatively, the cytotoxic genes, antisense sequences, gene products, retroviral vector constructs or viral particles , may also be administered to a warm-blooded animal by a variety of other methods. Representative examples include transfection by various physical methods, such as lipofection (Felgner et al., Proc. Natl. Acad Sci. USA 84:7413-7417, 1989), direct DNA injection (Acsadi et al., Nature 352:815-818, 1991); microprojectile bombardment (Williams et al., PNAS 88:2726-2730, 1991); liposomes of several types (*see e.g.,* Wang et al., PNAS 84:7851-7855, 1987); CaPO₄ (Dubensky et al., PNAS 81:7529-7533, 1984); DNA ligand (Wu et al, J. of Biol. Chem. 264:16985-16987, 1989); administration of nucleic acids alone (WO 90/11092); or administration of DNA linked to killed adenovirus (Curiel et al., Hum. Gene Ther. 3: 147-154, 1992).

Retroviral particles (or retroviral vector constructs alone) may be utilized in order to directly treat pathogenic agents such as a tumor. The retroviral particles or retroviral vector constructs described above may be directly administered to a tumor, for example, by direct injection into several different locations within the body of tumor. Alternatively, arteries which serve a tumor may be identified, and the vector injected into such an artery, in order to deliver the vector directly into the tumor. A tumor which has a necrotic center may be aspirated, and the vector injected directly into the now empty center of the tumor. The retroviral vector construct may be directly administered to the surface of the tumor, for example, by application of a topical pharmaceutical composition containing the retroviral vector construct, or preferably, a recombinant retroviral particle.

Methods may be provided for inhibiting the growth of a selected tumor in a warm-blooded animal, comprising the steps of (a) removing tumor cells associated with the selected tumor from a warm-blooded animal, (b) infecting the removed cells with a retroviral vector construct which directs the expression of at least one anti-tumor agent, and (c) delivering the infected cells to a warm-blooded animal, such that the growth of the selected tumor is inhibited by immune responses generated against the gene-modified tumor cell.

"Inhibiting the growth of a selected tumor" refers to either (I) the direct inhibition of tumor cell division, or (2) immune cell mediated tumor cell lysis, or both, which leads to a suppression in the net expansion of tumor cells. Inhibition of tumor growth by either of these two mechanisms may be readily determined by one of ordinary skill in the art based upon a number of well known methods (*see* U.S. Serial No. 08/032,846). Examples of compounds or molecules which act as anti-tumor agents include immune accessory molecules, cytotoxic genes, and antisense sequences as discussed above (*see also* U.S. Serial No. 08/032,846).

Cells may be removed from a variety of locations including, for example, from a selected tumor. In addition a vector construct may be inserted into non-tumorigenic cells, including for example, cells from the skin (dermal fibroblasts), or from the blood (*e.g*., peripheral blood leukocytes). If desired, particular fractions of cells such as a T cell subset or stem cells may also be specifically removed from the blood (*see,* for example, PCT WO 91/16116, an application entitled "Immunoselection Device and Method"). Vector constructs may then be contacted with the removed cells utilizing any of the above-described techniques, followed by the return of the cells to the warm-blooded animal, preferably to or within the vicinity of a tumor. Subsequent to removing tumor cells from a warm-blooded animal, a single cell suspension may be generated by, for example, physical disruption or proteolytic digestion. In addition, division of the cells may be increased by addition of various factors such as melanocyte stimulating factor for melanomas or epidermal growth factor for breast carcinomas, in order to enhance uptake, genomic integration and expression of the recombinant viral vector.

It should be understood that the removed cells may not only be returned to the same animal, but may also be utilized to inhibit the growth of selected tumor cells in another, allogeneic, animal. In such a case it is generally preferable to have histocompatibility matched animals (although not always, *see, e.g.,* Yamamoto et al., "Efficacy of Experimental FIV Vaccines," 1st International Conference of FIV Researchers, University of California at Davis, September 1991).

The above-described methods may additionally comprise the steps of depleting fibroblasts or other non-contaminating tumor cells subsequent to removing tumor cells from a warm-blooded animal, and/or the step of inactivating the cells, for example, by irradiation.

As noted above, several anti-tumor agents may be administered either concurrently or sequentially, in order to inhibit the growth of a selected tumor in accordance with the methods of the present invention. For example, an anti-tumor agent such as γ-IFN may be co-administered or sequentially administered to a warm-blooded animal along with other anti-tumor agents such as IL-2, or IL-12, in order to inhibit or destroy a pathogenic agent. Such therapeutic compositions may be administered directly utilizing a single vector construct which directs the expression of at least two anti-tumor agents, or, expressed from independent vector constructs. Alternatively, one anti-tumor agent (*e.g*., γ-IFN) may be administered utilizing a vector construct, while other tumor agents (*e.g.,* IL-2) are administered directly (*e.g*., as a pharmaceutical composition intravenously).

Retroviral vector constructs which deliver and express both a γ-IFN gene and another gene encoding IL-2, may be administered to the patient. In such constructs, one gene may be expressed from the retrovector LTR and the other may utilize an additional transcriptional promoter found between the LTRs, or may be expressed as a polycistronic mRNA, possibly utilizing an internal ribosome binding site. After *in vivo* gene transfer, the patient's immune system is activated due to the expression of γ-IFN. Infiltration of the dying tumor with inflammatory cells, in turn, increases immune presentation and further improves the patient's immune response against the tumor.

Methods may be provided for generating an immune response against an immunogenic portion of an antigen, in order to prevent or treat a disease (*see*, *e.g.,* U.S. Serial Nos. 08/104,424; 08/102,132, 07/948,358; 07/965,084), for suppressing graft rejection, (*see* U.S. Serial No. 08/116,827), for suppressing an immune response (*see* U.S. Serial No. 08/11b,828), and for suppressing an autoimmune response (*see* U.S. Serial No. 08/116,983).

As will be understood by one of ordinary skill in the art given the disclosure provided herein, any of the retroviral vector constructs described herein may be delivered not only as a recombinant viral particle, but as direct nucleic acid vectors. Such vectors may be delivered utilizing any appropriate physical method of gene transfer, including for example, those which have been discussed above.

The following examples are offered by way of illustration, and not by way of limitation.

### EXAMPLE 1

### CONSTRUCTION OF RETROVECTOR BACKBONES

### A. Preparation of a Retroviral vector construct That Does Not Contain an Extended Packaging Sequence (ψ)

This example describes the construction of a retroviral vector construct using site-specific mutagenesis. Within this example, a MoMLV retroviral vector construct is prepared wherein the packaging signal "ψ" of the retrovector is terminated at basepair 617 of SEQ ID NO: 1, thereby eliminating the ATG start of *gag.* Thus, no crossover can occur between the retroviral vector construct and the *gag*/*pol* expression cassette which is described below in Example 3.

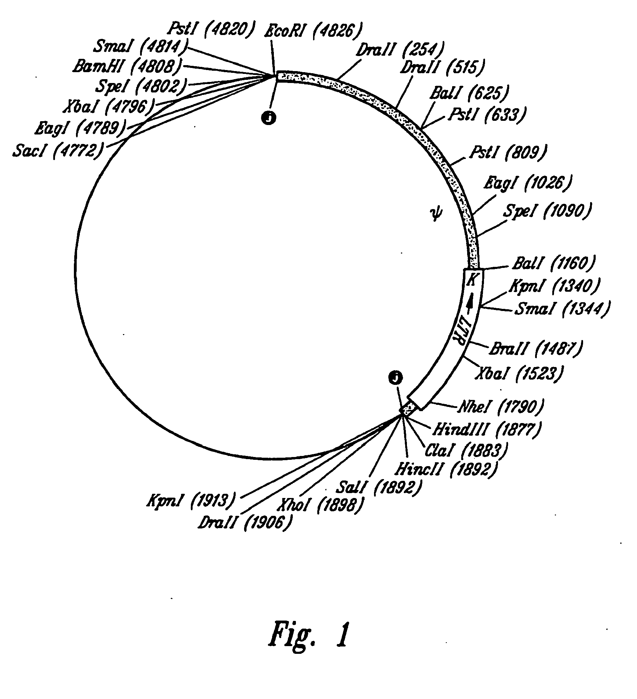

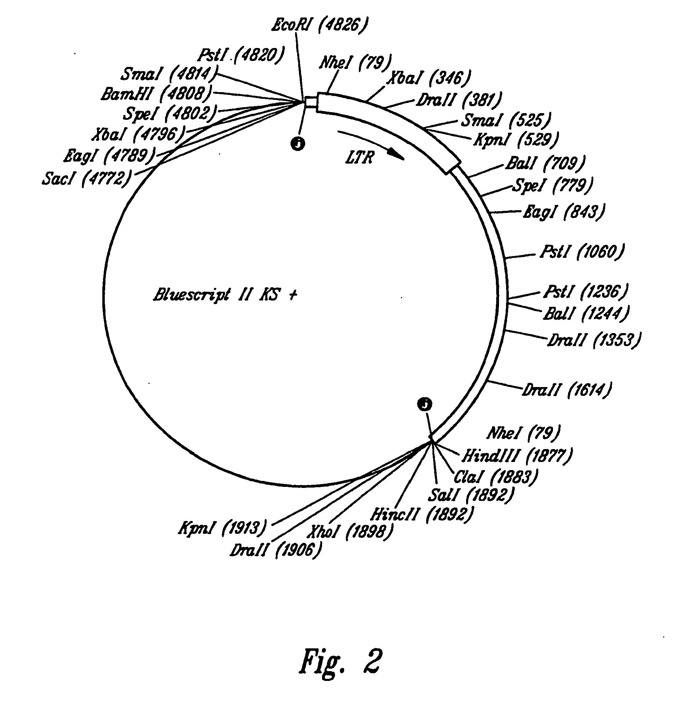

Briefly, pMLV-K (Miller, J. Virol 49:214-222, 1984 - an infectious clone derived from pMLV-1 Shinnick et al., Nature, 293:543-548, 1981) is digested with *Eco*57I, and a 1.9kb fragment is isolated. (*Eco*571 cuts upstream from the 3' LTR, thereby removing all *env* coding segments from the retroviral vector construct.) The fragment is then blunt ended with T4 polymerase (New England Biolabs), and all four deoxynucleotides, and cloned into the *Eco*RV site of phagemid pBluescript II KS+ (Stratagene, San Diego, Calif.). This procedure yields two constructs, designated pKS2+Eco57I-LTR(+) (Figure 1) and pKS2+Eco57I-LTR(-) (Figure 2), which are screened by restriction analysis. When the (+) single stranded phagemid is generated, the sense sequence of MoMLV is isolated.

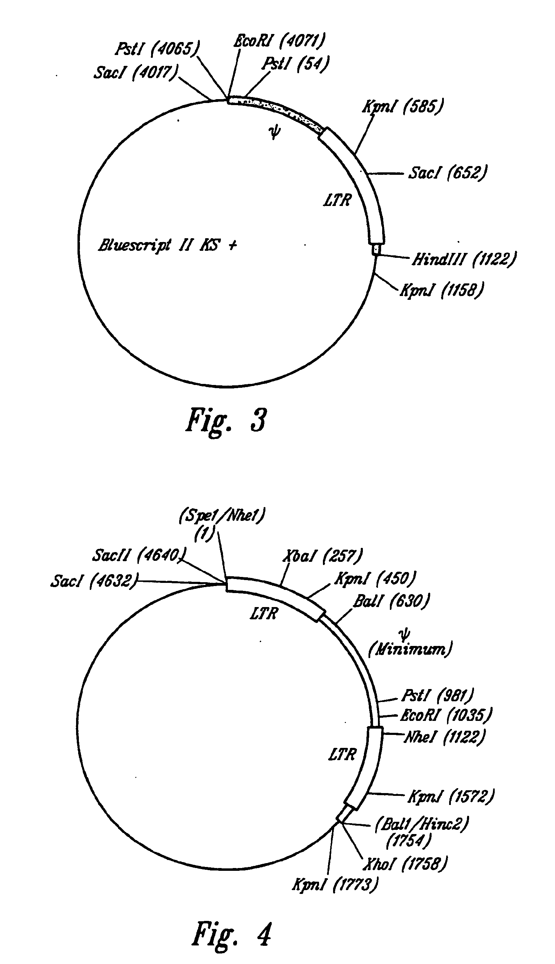

A new *Eco*RI site is then created in construct pKS2+*Eco*57I-LTR(+) in order to remove the ATG start codon of *gag.* In particular, an *Eco*RI site is created using the single stranded mutagenesis method of Kunkle (PNAS 82:488, 1985). pKS2+*Eco*57I-LTR(+) is a pBluescript™ II + phagemid (Strategene, San Diego, Calif.) containing an *Eco*57I fragment from pMLV-K. It includes the MoMLV LTR and downstream sequence to basepair 1378. When single stranded phagemid is generated the sense sequence of MoMLV is isolated. The oligonucleotide, 5'-GGT AAC AGT CTG GCC CGA ATT CTC AGA CAA ATA CAG (SEQ ID NO: 2), is created and used to generate an *Eco*RI site at basepairs 617-622. This construct is designated pKS2+LTR-EcoRI (Figure 3).

### B. Substitution of Nonsense Codons in the Extended Packaging Sequence (ψ+)

This example describes modification of the extended packaging signal (ψ+) by site-specific mutagenesis. In particular, the modification will substitute a stop codon, TAA, at the normal ATG start site of *gag* (position 631-633 of SEQ ID NO: 1), and an additional stop codon TAG at position 637-639 of SEQ ID NO: 1.

Briefly, an *Eco*57I *- Eco*RI fragment (MoMLV basepairs 7770 to approx. 1040) from pN2 (Amentano et al., J. Virol. 61:1647-1650, 1987) is first cloned into pBluescript II KS+ phagemid at the *Sac*II and *Eco*RI sites (compatible). Single stranded phagemid containing antisense MoMLV sequence, is generated using helper phage M13K07 (Stratagene, San Diego, Calif.). The oligonucleotide 5'-CTG TAT TTG TCT GAG AAT TAA GGC TAG ACT GTT ACC AC (SEQ ID NO: 3) is synthesized, and utilize according to the method of Kunkle as described above, in order to modify the sequence within the ψ region to encode stop codons at nucleotides 631-633 and 637-639.

### C. Removal of Retroviral Packaging Sequence Downstream from the 3' LTR

Retroviral packaging sequence which is downstream from the 3' LTR is deleted essentially as described below. Briefly, pKS2+Eco57I-LTR(-) (Figure 2) is digested with *BaII* and *Hinc*II*,* and relegated excluding the *BalI* to *Hinc*II DNA which contains the packaging region of MoMLV.

### D. Construction of Vector Backbones

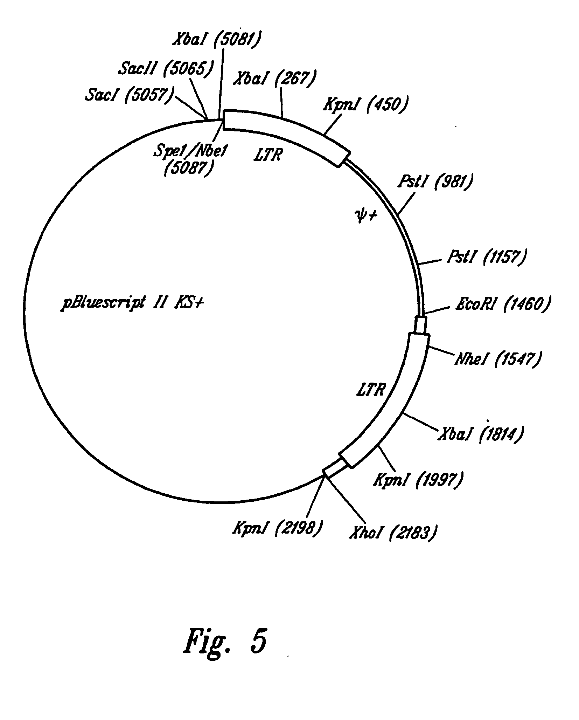

Constructs prepared in sections A and C above, or alternatively from sections B and C above, are combined with a plasmid vector as described below, in order to create a retrovector backbone containing all elements required *in cis,* and excluding all sequences of 8 nucleic acids or more contained in the retroviral portion of the *gag-pol* and *env* expression elements (*see* Examples 3 and 4).
1. Parts A and C are combined as follows: The product of A is digested with *Nhe*I and *Eco*RI, and a 1034 basepair fragment containing the LTR and minimal ψ is isolated. The fragment is ligated into the product of part C at the unique (compatible) restriction sites *Spe*I and *Eco*RI. The resultant construct is designated pR1 (Figure 4)
2. Parts B and C are combined as follows: The product of B is digested with *Nhe*I and *Eco*RI and a 1456 basepair fragment containing the LTR and modified ψ+ region is isolated. The fragment is ligated into the product of C at the unique (compatible) restriction sites *Spe*I and *Eco*RI. The resultant construct is designated pR2 (Figure 5).

### EXAMPLE 2

### INSERTION OF A GENE OF INTEREST INTO PR1 AND PR2

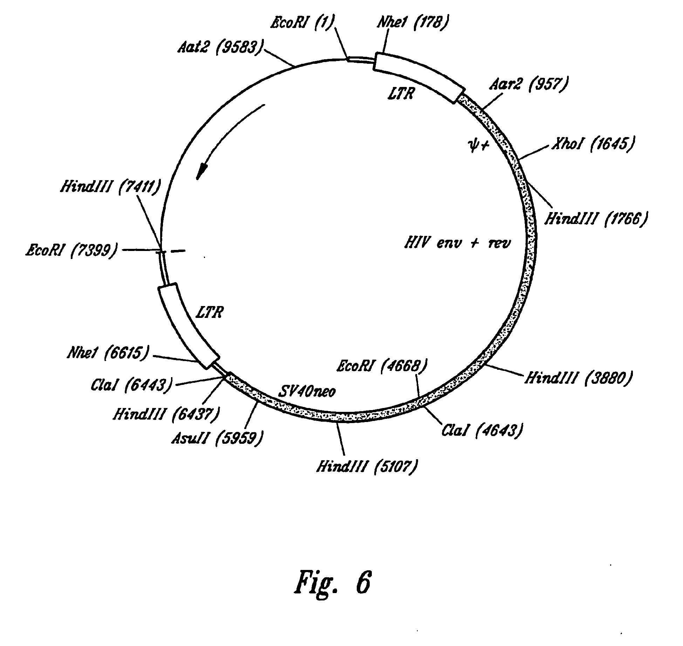

This example describes the insertion of a gene of interest, gp120, gp41, and rev along with a selectable marker into either pR1 or pR2. Briefly, the sequence encoding gp120, gp41 and rev is taken from construct pKT1 (Figure 6; *see also* Chada et al., J. Vir. 67:3409-3417, 1993); note that this vector is also referred to as N2HIBenv. In particular, pKT1 is first digested at the unique *Asu*II site (position 5959). The ends are blunted, and an *Xho* I linker is ligated at that site. (New England Biolabs). The construct is then digested with *Xho* I, and a 4314 bp fragment containing HIV envelope (gp120 and gp41), rev, SV40 early promoter and G418 resistance genes is isolated.

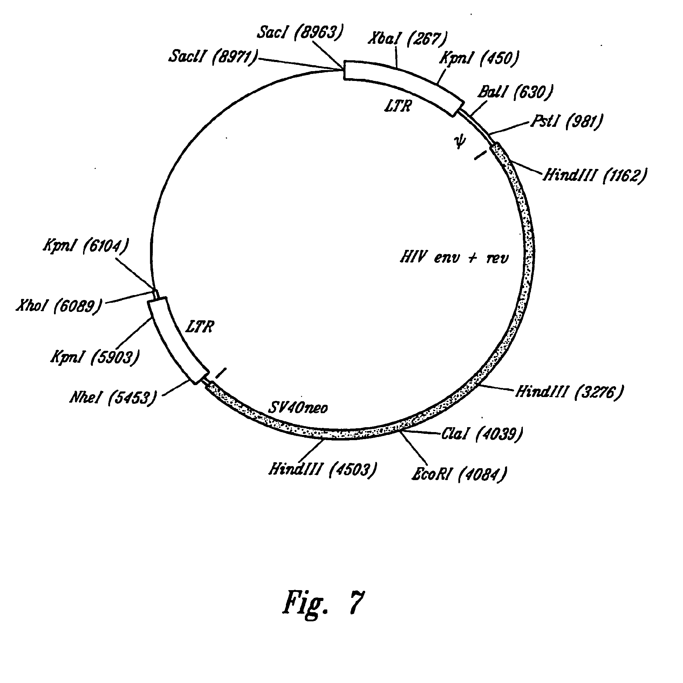

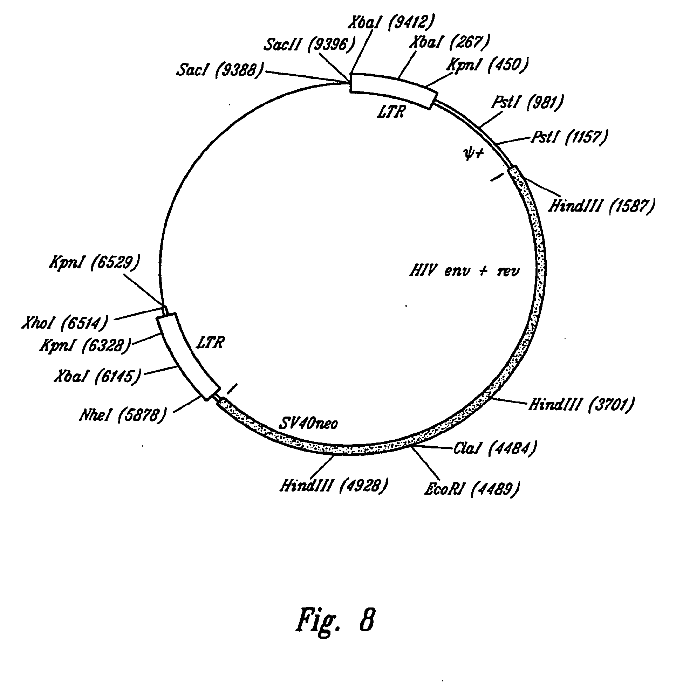

pR1 or pR2 is digested at the unique *Eco* R1 restriction site, blunted, and *Sal* I linkers (New England Biolabs) are ligated in. The 4314 bp KT1 fragment is then ligated into pR1 or pR2 at the new *Sal* I sites, and the correct orientation is determined *(see* Figures 7 and 8). In both of these constructs, (pR1-HTVenv and pR2-HIVenv) the HIV genes are expressed from the MLV LTR, and G418 resistance is expressed from the SV40 promoter.

### EXAMPLE 3

### CONSTRUCTION OF GAG-POL EXPRESSION CASSETTES

### A. Construction of an Expression Cassette Backbone, pHCMU-PA

A vector is first created in order to form the backbone for both the *gag*/*pol* and *env* expression cassettes. Briefly, pBluescript SK- phagemid (Stratagene, San Diego, Calif.; GenBank accession number 52324; referred to as "SK-") is digested with *Spe*I and blunt ended with Klenow. A blunt end *Dra*I fragment of SV40 (Fiers et al., "Complete nucleotide sequence of SV40 DNA" Nature 273:113-120, 1978) from DraI (bp 2366) to DraI (bp2729) is then inserted into SK-, and a construct isolated in which the SV40 late polyadenylation signal is oriented opposite to the LacZ gene of SK-. This construct is designated SK-SV40A.

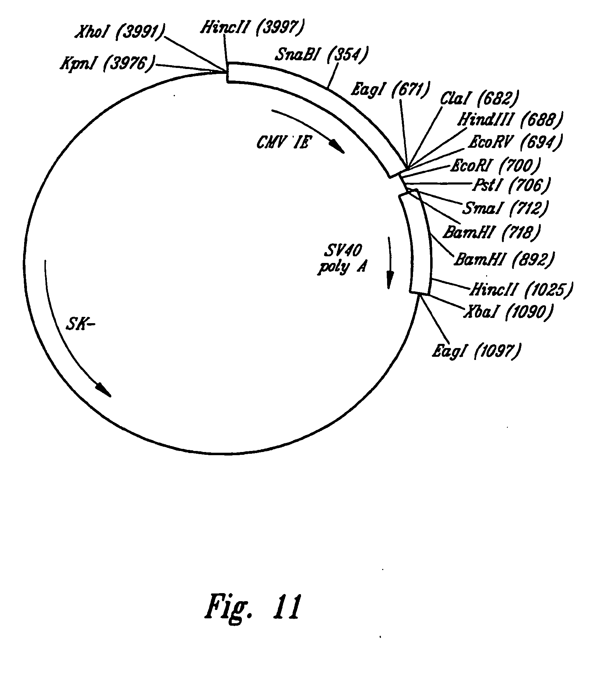

A Human Cytomegalovirus Major Immediate Early Promoter ("HCMV-IE"; Boshart et al., Cell 41:521-530, 1985) (*Hinc*II, bp 140, to *Eag*I, bp814) is isolated after digestion with *Hinc*II and *Eag*I, and the *Eag*I site blunt ended. The 674 blunt ended fragment is ligated into SK-SV40A. The final construct, designated pHCMV-PA is then isolated (*see* Figure 11). This construct contains the HCMV promoter oriented in opposite orientation to the LacZ gene, and upstream from the late polyadenylation signal of SV40.

### B. Creation of New Codons for the 5' Gag

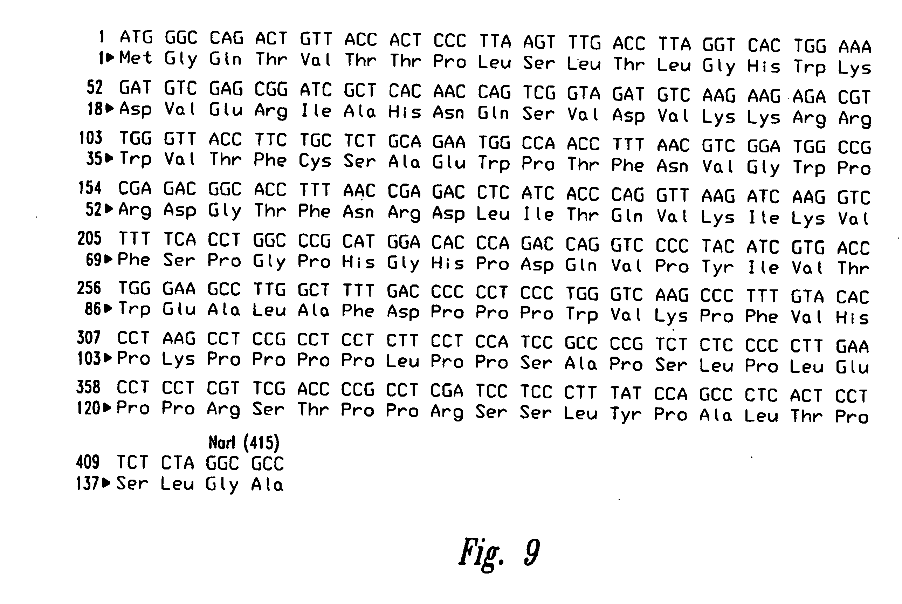

This example describes *gag*/*pol* expression cassettes that lack non-coding sequences upstream from the *gag* start, thereby reducing recombination potential between the *gag-pol* expression element and ψ+ sequence of a retroviral vector construct, and inhibiting co-packaging of the *gag-pol* expression element along with the retrovector. In order to construct such an expression cassette, 448 bp of DNA is synthesized with the following features: 5' ATATATATATATCGAT(ClaI site)ACCATG(start codon, position 621) (SEQ ID NO: 4), followed by 410 bp encoding 136+ amino acid residues using alternative codons *(see* Figures 9 and 10), followed by GGCGCC(*Nar*I site)AAACCTAAAC 3' (SEQ ID NO: 5).

Briefly, each of oligos 15 through 24 (set forth below in Table 1) are added to a PCR reaction tube such that the final concentration for each is 1 µM. Oligos 25 and 26 are added to the tube such that the final concentration for each is 3 µM. 1.2 µL of 2.5 mM stock deoxynucleotide triphosphates (dG, dA, dT, dC) are added to the tube. 5 µL of 10X PCR buffer (Perkin Elmer). Water is added to a final volume of 50 µL. Wax beads are added and melted over the aqueous layer at 55°C and then cooled to 22°C. A top aqueous layer is added as follows: 5 µL 10X PCR buffer, 7.5 µL dimethylsulfoxide, 1.5 µL Taq polymerase (Perkin-Elmer) and 36 µL water. Forty cycles of PCR are then performed as follows: 94°C, 30 seconds; 56°C, 30 seconds; and 72°C, 30 seconds. The PCR product is stored at -20°C until assembly of the *gag*/*pol* expression cassette.

**Table 1**

| **SEQ. ID. No.** | **Sequence** |
|---|---|
| 15 | |
| 16 | |
| 17 | |
| 18 | |
| 19 | |
| 20 | |
| 21 | |
| 22 | |
| 23 | |
| 24 | |
| 25 | 5' ATA TAT ATA TAT CGA TAC C 3' |
| 26 | 5' GTT TAG GTT TGG CGC CGA GG 3' |

### C. Creation of a New 3' End for Pol

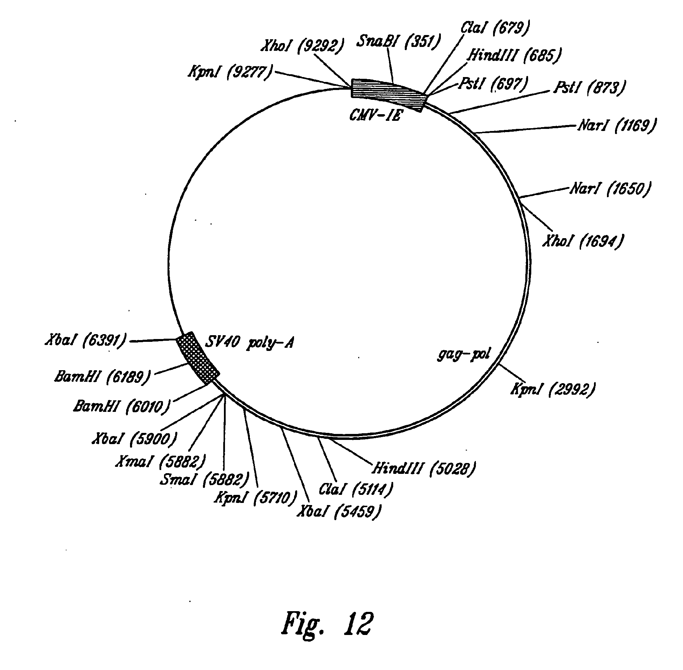

In order to prepare a *gag*/*pol* expression cassette which expresses full length *gaglpol, pCMVgag*/*pol* is constructed. Briefly, MoMLV sequence from *Pst*1 (BP567) to *Nhe1* (bp 7847) is cloned into the *Pst*1*-Xba*1 sites of pUC19 (New England Biolabs). The resultant intermediate is digested with *Hin*dIII and *Xho*I, and a 1008 bp fragment containing the *gag* leader sequence is isolated. The same intermediate is also digested with *XhoI* and *ScaI,* and a 4312 bp fragment containing the remaining *gag* and *pol* sequences is isolated. The two isolated fragments are then cloned into the HindIII and *SmaI* sites of pHCMV-PA, described above. The resultant construct, designated CMV *gag*/*pol* (Figure 12) expresses MoMLV *gag* and *pol* genes.

In order to truncate the 3' end of the *pol* gene found in pCMV *gag-pol,* a 5531 basepair *SnaBI - XmaI* fragment containing a portion of the CMV IE promoter and all of *gag-pol* except the final 28 codons, is isolated from pCMV *gag-pol.* This fragment is cloned into the *Sn*aBI and *Xma*I sites of pHCMV-PA. This construct expresses five new amino acids at the carboxy-terminus (Ser-Lys-Asn-Tyr-Pro) (SEQ ID NO: 6) (pCMV gpSma).

Alternatively, these five amino acids may be eliminated by digesting pCMVgp *Sma*I with *Sma*I and adding an *Nhe*I (termination codons in three phases) linker (5' - CTA GCT AGC TAG SEQ ID NO: 14; New England Biolabs) at the end of the truncated *pol* sequence. This construct is designated pCMV gp Nhe. Both of these constructs eliminates potential crossover between *gaglpol* and *env* expression cassettes.

### D. Gag-Pol Expression Cassette

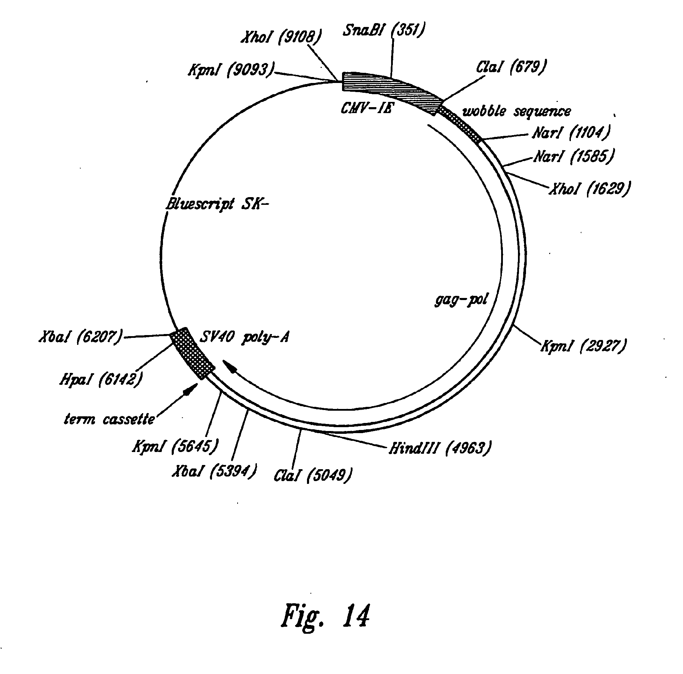

Parts B and C from above are combined to provide an expression vector containing a CMV IE promoter, *gag-pol* sequence starting from the new *Cla*I site (followed by ACC ATG and 412 bp of alternative or "wobble" *gag* coding sequence) and terminating at the *Sma*I site (MoMLV position 5750) followed by an SV40 polyadenylation signal, essentially as described below. Briefly, the approximately 451 bp double stranded wobble fragment from part A is ligated into pCR™II TA cloning vector (Invitrogen Corp.). The wobble PCR product naturally contains a 3' A-overhang at each end, allowing for cloning into the 3' T-overhang of pCR™II. The 422 bp *Cla*I *-Nar*I wobble fragment from the pCR™II clone is removed and is ligated into the *Cla*I (Position 679, Figure pCMV gp Sma) and *Nar*I (Position 1585) sites of pCMVgp Smal (Part B) (or pCMV gp Nhe). (The *Cla*I site at position 5114 is methylated and not cut with *Cla*I)*.* The product of that ligation is digested with *Narl,* and the MLV-K *Nar*I fragment (positions 1035 to 1378) is inserted (SEQ ID NO: 1). This construct is designated pCMVgp -X (Figure 14).

### EXAMPLE 4

### CONSTRUCTION OF ENV EXPRESSION CASSETTES

### A. Creation of a New 5' Eagl Restriction Site

Starting with an *Eag*I*- Eco*RI 626 bp subfragment from a 4070A amphotropic envelope (Chattopodhyay et al., J. Vir. 39:777, 1981; GenBank accession # MLV4070A, and #MLVENVC; SEQ ID NO: 12) cloned in a pBluescript II Ks+ vector (containing the start codon), site directed mutagenesis is performed upstream of the translation start site in order to change ACCATCCTCTGGACGGACATG... (SEQ ID NO: 7; positions 20- 40 of Genebank sequence # MLVENVC) to ACCCGGCCGTGGACGGACATG... (SEQ ID NO: 8) and create a new *Eag*I site at position 23. This modification allows cloning of the amphotropic envelope sequence into an expression vector eliminating upstream 4070A sequence homologous to the *gag-pol* expression element as described in Example 2A.

### B. Creation of a New 3' End for Env

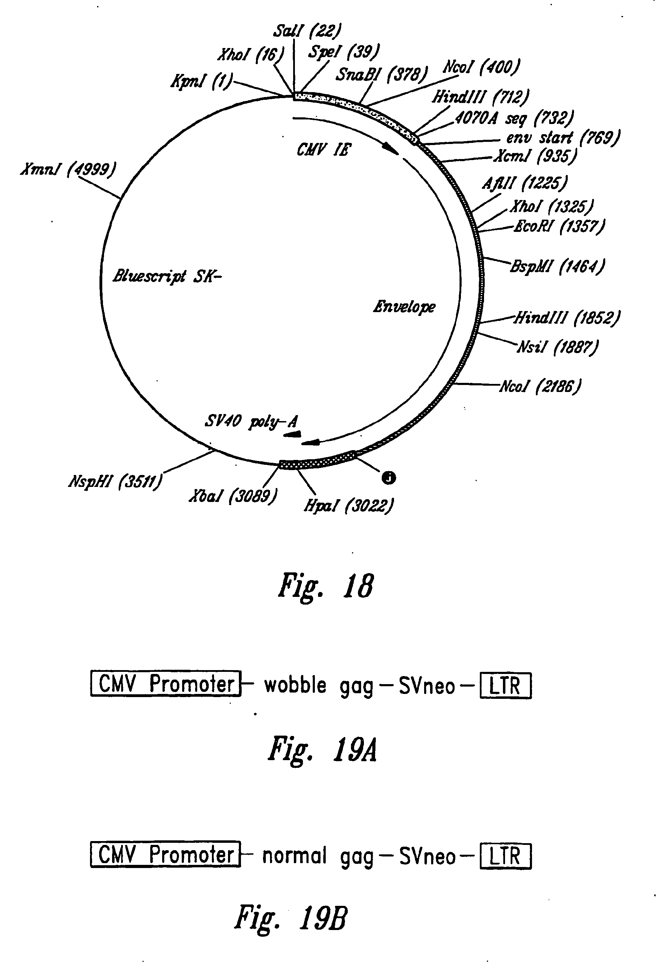

A new 3' end of the envelope expression element is created by terminating the sequence which encodes the R-peptide downstream from the end of the transmembrane region (p15E). Briefly, construct pHCMV-PA, described above, is first modified by digestion with *Not*I (position 1097), blunted and relegated to obliterate the overlapping Bluescript *Eag*I site at the same position. pCMV Envam-Eag-X-less is then constructed by digesting the modified pHCMV-PA with *Eag*I (position 671 and *Sma*I (position 712) and ligating in two fragments. The first is an *EagI-NcoI* fragment from 4070A (positions 1-1455) (SEQ ID NO: 12). The second is an MLV-K envelope fragment, *NcoI-Pvu*II (positions 7227-7747) (SEQ ID NO: 12). The resultant construct from the three-way ligation contains the HCMV promoter followed by the SU (GP70) coding sequence of the 4070A envelope, the TM (p15e) coding sequence of MoMLV, and sequence encoding 8 residues of the R-peptide. In addition, this envelope expression cassette (pCMV Env am-Eag-X-less) (Figure 18) shares no sequence with crossless retrovector backbones described in Example 1.

### C. Envelope Expression Element

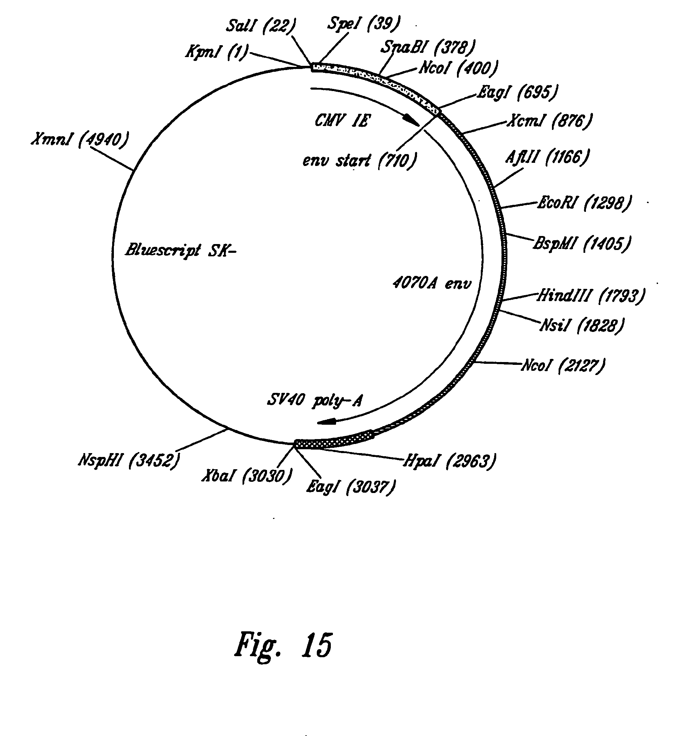

Parts A and B from above are combined to complete an amphotropic expression element containing the CMV promoter, 4070A SU, MoMLV TM and SV40 polyadenylation signal in a Bluescript SK- plasmid vector. This construct is called pCMVenv-X (Figure 15). Briefly, the construct described in part A with a new *Eag1* restriction site is digested with *Eag*I and *Xho*I, and a 571 bp fragment is isolated. pCMV Envam-Eag-X-less (from part B) is digested with *Kpn*I and *Eag*I and the 695 bp fragment is reserved. pCMV Envam-Eag-X-less (from part B) is digested with *Kpn*I and *XhoI* and the 4649 bp fragment is reserved. These two fragments are ligated together along with the 571 bp *Eag*I to *Xho*I fragment digested from the PCR construct from part A. pCMVenv-X shares no sequence with crossless retrovector backbones nor the *gag-pol* expression element pCMVgp-X.

### EXAMPLE 5

### FUNCTIONALITY TESTS FOR GAG-POL AND ENV EXPRESSION CASSETTES

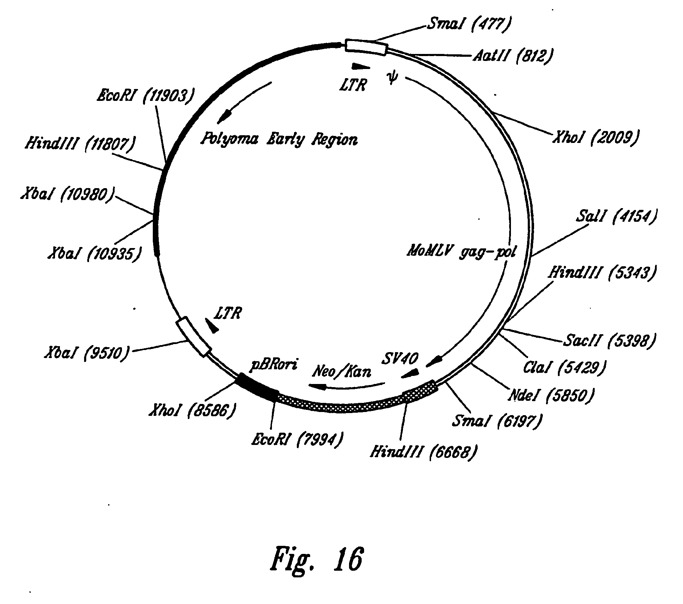

Rapid tests have been developed in order to ensure that the *gag-pol* and env expression cassettes are biologically active. The materials for these tests consist of a cell line used for transient expression (typically 293 cells, ATCC #CRL 1573), a target cell line sensitive to infection (typically HT 1080 cells, ATCC #CCL 121) and either pRgpNeo (Figure 16) or pLARNL (Emi et al., J. Virol 65:1202-1207, 1991). The two later plasmids express rescuable retrovectors that confer G418 resistance and also express *gag-pol,* in the case of RgpNeo or *env,* in the case of pLARNL. For convenience, the organization of RgpNeo (Figure 16) is set forth below.

In order to test expression cassettes such as pCMVgp-X for functionality of *gag*/*pol,* the plasmid is co-transfected with pLARNL at a 1:1 ratio into 293 cells. After 12 hours, the media is replaced with normal growth media. After an additional 24 hours, supernatant fluid is removed from the 293 cells, filtered through a 0.45 µm filter, and placed on HT 1080 target cells. Twenty-four hours after that treatment, the media is replaced with growth media containing 800 ug/ml G418. G418 resistant colonies are scored after one week. The positive appearance of colonies indicates that all elements are functional and active in the original co-transfection.

For convenience, the organization of RgpNeo (Figure 16) is set forth below:
Position 1 = left end of 5' LTR; Positions 1-6320 = MoMLV sequence from 5'LTR to Sca 1 restriction site; Positions 6321 - 6675 = SV40 early promoter; Positions 6676-8001 = Neo resistance gene from Tn 5 (including prokaryotic promoter); and Positions 8002 - 8606 = pBR origin of replication.

### EXAMPLE 6

### PACKAGING CELL LINE AND PRODUCER CELL LINE DEVELOPMENT

This example describes the production of packing and producer cell lines utilizing the above described retroviral vector constructs, *gag*/*pol* expression cassettes, and *env* expression cassettes, which preclude the formation of replication competent virus.

Briefly, for amphotropic MoMLV-based retroviral vector constructs, a parent cell line is selected which lacks sequences which are homologous to Murine Leukemia Viruses, such as the dog cell line D-17 (ATCC No. CCL 183). The *gaglpol* expression cassettes are then introduced into the cell by electroporation, along with a selectable marker plasmid such as DHFR (Simonsen et al., PNAS 80:2495-2499, 1983). Resistant colonies are then selected, expanded in 6 well plates to confluency, and assayed for expression of gag/pol by Western Blots. Clones are also screened for the production of high titer vector particles after transduction with pLARNL.

The highest titer clones are then electroporated with an *env* expression cassette and a selectable marker plasmid such as hygromycin (*see* Gritz and Davies, Gene 25:179-188, 1983). Resistant colonies are selected, expanded in 6 well plates to confluency, and assayed for expression of env by Western Blots. Clones are also screened for the production of high titer vector particles after transduction with a retroviral vector construct.

Resultant packaging cell lines may be stored in liquid Nitrogen at 10 x 10⁶ cells per vial, in DMEM containing 10% irradiated Fetal Bovine Serum, and 8% DMSO. Further testing may be accomplished in order to confirm sterility, and lack of helper virus production. Preferably, both an S+L- assay and a *Mus dunni* marker rescue assay should be performed in order to confirm a lack of helper virus production.

In order to construct a producer cell line, retroviral vector construct as described above in Example 1 is electroporated into a xenotropic packaging cell line made utilizing the methods described above. After 24-48 hours, supernatant fluid is removed from the xenotropic packaging cell line, and utilized to transduce a second packaging cell line, thereby creating the final producer cell line.

### EXAMPLE 7

### HELPER DETECTION ASSAY COCULTIVATION, AND MARKER RESCUE

This example describes a sensitive assay for the detection of replication competent retrovirus ("RCR"). Briefly, 5 x 10⁵ vector-producing cells are cocultivated with an equal number of *Mus dunni* cells (Lander and Chattopadhyay, J. Virol. 52:695, 1984). *Mus dunni* cells are particularly preferred for helper virus detection because they are sensitive to nearly all murine leukemia-related viruses, and contain no known endogenous viruses. At three, six, and nine days after the initial culture, the cells are split approximately 1 to 10, and 5 x 10⁵ fresh *Mus dunni* cells are added. Fifteen days after the initial cocultivation of *Mus dunni* cells with the vector-producing cells, supernatant fluid is removed from cultures, filtered through a 0.45 µm filter, and subjected to a marker rescue assay.

Briefly, culture fluid is removed from a MdH tester cell line *(Mus dunni* cells containing pLHL (a hygromycin resistance marker retroviral vector; see Palmer et al., PNAS 84(4):1055-1059, 1987) and replaced with the culture fluid to be tested. Polybrene is added to a final concentration of 4 µg/ml. On day 2, medium is removed and replaced with 2 ml of fresh DMEM containing 10% Fetal Calf Serum. On day 3, supernatant fluid is removed, filtered, and transferred to HT1080 cells. Polybrene is added to a final concentration of 4µg/ml. On day 4, medium in the HT1080 cells is replaced with fresh DMEM containing 10% Fetal Calf Serum, and 100 µg/ml hygromycin. Selection is continued on days 5 through 20 until hygromycin resistant colonies can be scored, and all negative controls (*e*.*g*., mock infected MdH cells) are dead.

### EXAMPLE 8

### ASSAY FOR ENCAPSIDATION OF WOBBLE RNA SEQUENCE

This example describes a sensitive assay for the detection of encapsidation of RNA from constructs containing wobble or normal gag sequence. Briefly, a fragment of DNA from a "wobble" *gag*/*pol* expression cassette (Example 3), containing the CMV promoter and gag sequence to the *Xho*I site (MoMLV position 1561) is ligated to a SV40 neo-3' LTR DNA fragment from N2 (Armentano et al., *supra)* or KT-3 *(see* WO 91/02805 or WO 92/05266). This construct is diagrammatically illustrated in Figure 19A, and is not expected to be encapsidated in packaging cell lines such as DA or HX (s*ee* WO 92/05266) because it lacks a 5' LTR and primer binding site.

A second construct is also made, similar to the first except that the wobble sequence is replaced by normal *gag* sequence. Similar to the first construct, the RNA transcribed from this DNA is not expected to be encapsidated. This construct is diagrammatically illustrated in Figure 19B.

The above constructs are separately transfected into a packaging cell line. The culture is then assayed for the ability to generate transducible G418-resistant retrovector. Neither construct results in transducible vector.

Cell cultures containing the above constructs are then transduced with the retrovector LHL (see Example 7). The cell cultures, after selection, will now generate retrovector conferring hygromycin resistance to target cells. Further, if co-encapsidation is allowed by interaction between LHL RNA and the transcripts from the above constructs, statistically significant RT-mediated recombination can occur resulting in the transfer of G418 resistance to target cells.

From the foregoing, it will be appreciated that, although specific embodiments of the invention have been described herein for purposes of illustration, various modifications may be made without deviating from the scope of the invention.

## Claims

1. A packaging cell line, comprising a *gag*/*pol* expression cassette comprising a promoter operably linked to a *gag*/*pol* gene, and a polyadenylation sequence, wherein the 5' terminal end of said *gag*/*pol* gene has been modified to contain at least 25 codons which are degenerate for *gag* and an env expression cassette.

2. The packaging cell line according to claim 1 wherein a 3' terminal end of said *gag*/*pol* gene has been deleted without affecting the biological activity of integrase.

3. The packaging cell line according to claim 2 wherein the sequence encoding the *gaglpol* gene is a Murine Leukemia Virus MoMLV sequence encoded by nucleotides 621 through 5834 of SEQ ID NO: 1 and wherein in the *gag*/*pol* expression cassette said 3' terminal end has been deleted downstream of nucleotide 5750 of Sequence ID NO. 1.

4. The packaging cell line according to any one of claims 1 to 3 wherein said 5' terminal end of said *gag*/*pol* gene contains less than 20 consecutive nucleotides which are identical to a sequence found in a retroviral packaging signal.

5. The packaging cell line according to any one of claims 1 to 4 wherein in the *gag*/*pol* expression cassette said promoter is a heterologous promoter.

6. The packaging cell line according to claim 5 wherein in the *gag*/*pol* expression cassette said promoter is selected from the group consisting of CMV IE, the HSVTK promoter, RSV promoter, Adenovirus major-later promoter and the SV40 promoter.

7. The packaging cell line according to any one of claims 1 to 6 wherein in the *gag*/*pol* expression cassette said polyadenylation sequence is a heterologous polyadenylation sequence.

8. The packaging cell line according to claim 7 wherein said heterologous polyadenylation sequence is selected from the group consisting of the SV40 late poly A signal and the SV40 early poly A signal.

9. The packaging cell line according to claim 1 wherein the *env* expression cassette comprises a promoter linked to an *env* gene, and a polyadenylation sequence and wherein a 3' terminal end of the *env* gene has been deleted without effecting the biological activity of *env*.

10. A producer cell line comprising a packaging cell line according to any one of claims 1 to 9 and a retroviral vector construct.

11. A method of producing a packaging cell, comprising:
(a) introducing a *gaglpol* expression cassette as defined in any one of claims 1 to 9 into an animal cell;
(b) selecting a cell containing a *gag*/*pol* expression cassette which expresses high levels of *gag*/*pol;*
(c) introducing an env expression cassette into said selected cell; and
(d) selecting a cell which expresses high levels *of env,* and thereby producing said packaging cell.

12. A method of producing recombinant retroviral particles, comprising introducing a retroviral vector construct into packaging cell line produced by a method according to claim 11.

## Patentansprüche

1. Verpackungszelllinie, umfassend eine einen mit einem gag/pol-Gen in operativer Verknüpfung stehenden Promotor und eine Polyadenylierungssequenz umfassende *gag*/*pol*-Expressionskassette, wobei das 5 '-terminale Ende des *gag*/*pol*-Gens so modifiziert wurde, dass es wenigstens 25 Codons, die für *gag* degeneriert sind, sowie eine env-Expressionskassette enthält.

2. Verpackungszelllinie nach Anspruch 1, wobei ein 3'-terminales Ende des *gag*/*pol*-Gens deletiert wurde, ohne sich auf die biologische Aktivität von Integrase auszuwirken.

3. Verpackungszelllinie nach Anspruch 2, wobei es sich bei der für das gag/pol-Gen codierenden Sequenz um eine von Nukleotiden 621 bis 5834 der SEQ ID NO: 1 codierte Sequenz des Maus-Leukämie-Virus MoMLV handelt und wobei in der gag/pol-Expressionskassette das 3`-terminale Ende stromabwärts von Nukleotid 5750 der Sequenz ID NO. 1 deletiert wurde.

4. Verpackungszelllinie nach einem der Ansprüche 1 bis 3, wobei das 5`-terminale Ende des *gag*/*pol-*Gens weniger als 20 aufeinanderfolgende Nukleotide enthält, die mit einer in einem retroviralen Verpackungssignal angetroffenen Sequenz identisch sind.

5. Verpackungszelllinie nach einem der Ansprüche 1 bis 4, wobei es sich bei dem Promotor in der gag/pol-Expressionskassette um einen heterologen Promotor handelt.

6. Verpackungszelllinie nach Anspruch 5, wobei der Promotor in der gag/pol-Expressionskassette aus der aus CMV IE, dem HSVTK-Promotor, RSV-Promotor, Major-later-Adenoviruspromotor und dem SV40-Promotor bestehenden Gruppe ausgewählt ist.

7. Verpackungszelllinie nach einem der Ansprüche 1 bis 6, wobei es sich bei der Polyadenylierungssequenz in der gag/pol-Expressionskassette um eine heterologe Polyadenylierungssequenz handelt.

8. Verpackungszelllinie nach Anspruch 7, wobei die Polyadenylierungssequenz aus der aus dem späten SV40-Poly-A-Signal und dem frühen SV40-Poly-A-Signal bestehenden Gruppe ausgewählt ist.

9. Verpackungszelllinie nach Anspruch 1, wobei die env-Expressionskassette einen mit einem env-Gen verknüpften Promotor und eine Polyadenylierungssequenz umfasst und wobei ein 3`-terminales Ende des env-Gens deletiert wurde, ohne sich auf die biologische Aktivität von env auszuwirken.

10. Produktionszelllinie, umfassend eine Verpackungszelllinie nach einem der Ansprüche 1 bis 9 sowie ein retrovirales Vektorkonstrukt.

11. Verfahren zur Herstellung einer Verpackungszelle, bei dem man:
(a) eine *gag*/*pol*-Expressionskassette mit der in einem der Ansprüche 1 bis 9 angegebenen Bedeutung in eine Tierzelle einführt;
(b) eine eine *gag*/*pol*-Expressionskassette enthaltende Zelle, die hohe *gag*/*pol*-Niveaus exprimiert, auswählt;
(c) eine env-Expressionskassette in die ausgewählte Zelle einführt; und
(d) eine Zelle, die hohe *env*-Niveaus exprimiert, auswählt und damit die Verpackungszelle herstellt.

12. Verfahren zur Herstellung rekombinanter Retroviruspartikel, bei dem man ein retrovirales Vektorkonstrukt in eine mit einem Verfahren nach Anspruch 11 hergestellte Verpackungszelllinie einführt.

## Revendications

1. Lignée cellulaire d'encapsidation, comprenant une cassette d'expression *gag*/*pol* comprenant un promoteur fonctionnellement lié à un gène *gag*/*pol* et une séquence de polyadénylation, dans laquelle l'extrémité 5'-terminale dudit gène *gag*/*pol* a été modifiée de façon à contenir au moins 25 codons qui sont dégénérés pour *gag* et une cassette d'expression *env.*

2. Lignée cellulaire d'encapsidation selon la revendication 1, dans laquelle une extrémité 3'-terminale dudit gène *gag*/*pol* a été délétée sans affecter l'activité biologique de l'intégrase.

3. Lignée cellulaire d'encapsidation selon la revendication 2, dans laquelle la séquence codant pour le gène *gag*/*pol* est une séquence MoMLV du virus de la leucémie murine, codée par les nucléotides 621 à 5834 de SEQ ID NO:1, et dans laquelle, dans la cassette d'expression *gag*/*pol*, ladite extrémité 3'-terminale a été délétée en aval du nucléotide 5750 de la séquence SEQ ID NO:1.

4. Lignée cellulaire d'encapsidation selon l'une quelconque des revendications 1 à 3, dans laquelle ladite extrémité 5'-terminale dudit gène *gag*/*pol* contient moins de 20 nucléotides consécutifs qui sont identiques à une séquence trouvée dans un signal d'encapsidation de rétrovirus.

5. Lignée cellulaire d'encapsidation selon l'une quelconque des revendications 1 à 4, dans laquelle, dans la cassette d'expression *gag*/*pol,* ledit promoteur est un promoteur hétérologue.

6. Lignée cellulaire d'encapsidation selon la revendication 5, dans laquelle, dans la cassette d'expression *gag*/*pol,* ledit promoteur est choisi dans le groupe consistant en le CMV IE, le promoteur HSVTK, le promoteur RSV, le promoteur tardif majeur de l'adénovirus et le promoteur SV40.

7. Lignée cellulaire d'encapsidation selon l'une quelconque des revendications 1 à 6, dans laquelle, dans la cassette d'expression *gag*/*pol,* ladite séquence de polyadénylation est une séquence de polyadénylation hétérologue.

8. Lignée cellulaire d'encapsidation selon la revendication 7, dans laquelle ladite séquence de polyadénylation hétérologue est choisie dans le groupe consistant en le signal poly A tardif du SV40 et le signal poly A précoce du SV40.

9. Lignée cellulaire d'encapsidation selon la revendication 1, dans laquelle la cassette d'expression *env* comprend un promoteur lié à un gène *env* et une séquence de polyadénylation, et dans laquelle une extrémité 3'-terminale du gène *env* a été délétée sans affecter l'activité biologique de l'env.

10. Lignée cellulaire productrice comprenant une lignée cellulaire d'encapsidation selon l'une quelconque des revendications 1 à 9 et une construction de vecteur rétroviral.

11. Procédé de production d'une cellule d'encapsidation, comprenant :
(a) l'introduction, dans une cellule animale, d'une cassette d'expression *gag*/*pol* telle que définie dans l'une quelconque des revendications 1 à 9 ;
(b) la sélection d'une cellule contenant une cassette d'expression *gag*/*pol* qui exprime des niveaux élevés de *gaglpol ;*
(c) l'introduction d'une cassette d'expression *env* dans ladite cellule sélectionnée ; et
(d) la sélection d'une cellule qui exprime des niveaux élevés de l'env, et produisant de ce fait ladite cellule d'encapsidation.

12. Procédé de production de particules rétrovirales recombinantes, comprenant l'introduction d'une construction de vecteur rétroviral dans une lignée cellulaire d'encapsidation produite par un procédé selon la revendication 11.


### SEQUENCE LISTING

(1) GENERAL INFORMATION:
   (i) APPLICANT: Viagene, Inc.
   (ii) TITLE OF INVENTION: CROSSLESS RETROVIRAL VECTORS
   (iii) NUMBER OF SEQUENCES: 26
   (iv) CORRESPONDENCE ADDRESS:
      (A) ADDRESSEE: Seed & Berry
      (B) STREET: 6300 Columbia Center: 701 Fifth Avenue
      (C) CITY: Seattle
      (D) STATE: Washington
      (E) COUNTRY: USA
      (F) ZIP: 98104
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0. Version #1.25
   (vi) CURRENT APPLICATION DATA:
      (A) APPLICATION NUMBER: PCT/US95/05789
      (B) FILING DATE: 09-MAY-1995
      (C) CLASSIFICATION:
   (viii) ATTORNEY/AGENT INFORMATION:
      (A) NAME: McMasters, David D
      (B) REGISTRATION NUMBER: 33.963
      (C) REFERENCE/DOCKET NUMBER: 930049.424PC
   (ix) TELECOMMUNICATION INFORMATION:
      (A) TELEPHONE: (206)622-4900
      (B) TELEFAX: (206)682-6031
(2) INFORMATION FOR SEQ ID NO:1:
   (i) SEQUENCE CHARACTERISTICS:
      (A) LENGTH: 8332 base pairs
      (B) TYPE: nucleic acid
      (C) STRANDEDNESS: single
      (D) TOPOLOGY: linear
   (xi) SEQUENCE DESCRIPTION: SEQ ID NO:1:
   GCGCCAGTCC TCCGATTGAC TGAGTCGCCC GGGTACCCGT GTATCCAATA AACCCTCTTG      60
   CAGTTGCATC CGACTTGTGG TCTCGCTGTT CCTTGGGAGG GTCTCCTCTG AGTGATTGAC     120
   TACCCGTCAG CGGGGGTCTT TCATTTGGGG GCTCGTCCGG GATCGGGAGA CCCCTGCCCA 180
   GGGACCACCG ACCCACCACC GGGAGGTAAG CTGGCCAGCA ACTTATCTGT GTCTGTCCGA 240
   TTGTCTAGTG TCTATGACTG ATTTTATGCG CCTGCGTCGG TACTAGTTAG CTAACTAGCT 300
   CTGTATCTGG CGGACCCGTG GTGGAACTGA CGAGTTCGGA ACACCCGGCC GCAACCCTGG 360
   GAGACGTCCC AGGGACTTCG GGGGCCGTTT TTGTGGCCCG ACCTGAGTCC AAAAATCCCG 420
   ATCGTTTTGG ACTCTTTGGT GCACCCCCCT TAGAGGAGGG ATATGTGGTT CTGGTAGGAG 480
   ACGAGAACCT AAAACAGTTC CCGCCTCCGT CTGAATTTTT GCTTTCGGTT TGGGACCGAA 540
   GCCGCGCCGC GCGTCTTGTC TGCTGCAGCA TCGTTCTGTG TTGTCTCTGT CTGACTGTGT 600
   TTCTGTATTT GTCTGAGAAT ATGGGCCAGA CTGTTACCAC TCCCTTAAGT TTGACCTTAG 660
   GTCACTGGAA AGATGTCGAG CGGATCGCTC ACAACCAGTC GGTAGATGTC AAGAAGAGAC 720
   GTTGGGTTAC CTTCTGCTCT GCAGAATGGC CAACCTTTAA CGTCGGATGG CCGCGAGACG 780
   GCACCTTTAA CCGAGACCTC ATCACCCAGG TTAAGATCAA GGTCTTTTCA CCTGGCCCGC 840
   ATGGACACCC AGACCAGGTC CCCTACATCG TGACCTGGGA AGCCTTGGCT TTTGACCCCC 900
   CTCCCTGGGT CAAGCCCTTT GTACACCCTA AGCCTCCGCC TCCTCTTCCT CCATCCGCCC 960
   CGTCTCTCCC CCTTGAACCT CCTCGTTCGA CCCCGCCTCG ATCCTCCCTT TATCCAGCCC 1020
   TCACTCCTTC TCTAGGCGCC AAACCTAAAC CTCAAGTTCT TTCTGACAGT GGGGGGCCGC 1080
   TCATCGACCT ACTTACAGAA GACCCCCCGC CTTATAGGGA CCCAAGACCA CCCCCTTCCG 1140
   ACAGGGACGG AAATGGTGGA GAAGCGACCC CTGCGGGAGA GGCACCGGAC CCCTCCCCAA 1200
   TGGCATCTCG CCTACGTGGG AGACGGGAGC CCCCTGTGGC CGACTCCACT ACCTCGCAGG 1260
   CATTCCCCCT CCGCGCAGGA GGAAACGGAC AGCTTCAATA CTGGCCGTTC TCCTCTTCTG 1320
   ACCTTTACAA CTGGAAAAAT AATAACCCTT CTTTTTCTGA AGATCCAGGT AAACTGACAG 1380
   CTCTGATCGA GTCTGTTCTC ATCACCCATC AGCCCACCTG GGACGACTGT CAGCAGCTGT 1440
   TGGGGACTCT GCTGACCGGA GAAGAAAAAC AACGGGTGCT CTTAGAGGCT AGAAAGGCGG 1500
   TGCGGGGCGA TGATGGGCGC CCCACTCAAC TGCCCAATGA AGTCGATGCC GCTTTTCCCC 1560
   TCGAGCGCCC AGACTGGGAT TACACCACCC AGGCAGGTAG GAACCACCTA GTCCACTATC 1620
   GCCAGTTGCT CCTAGCGGGT CTCCAAAACG CGGGCAGAAG CCCCACCAAT TTGGCCAAGG 1680
   TAAAAGGAAT AACACAAGGG CCCAATGAGT CTCCCTCGGC CTTCCTAGAG AGACTTAAGG 1740
   AAGCCTATCG CAGGTACACT CCTTATGACC CTGAGGACCC AGGGCAAGAA ACTAATGTGT 1800
   CTATGTCTTT CATTTGGCAG TCTGCCCCAG ACATTGGGAG AAAGTTAGAG AGGTTAGAAG 1860
   ATTTAAAAAA CAAGACGCTT GGAGATTTGG TTAGAGAGGC AGAAAAGATC TTTAATAAAC 1920
   GAGAAACCCC GGAAGAAAGA GAGGAACGTA TCAGGAGAGA AACAGAGGAA AAAGAAGAAC 1980
   GCCGTAGGAC AGAGGATGAG CAGAAAGAGA AAGAAAGAGA TCGTAGGAGA CATAGAGAGA 2040
   TGAGCAAGCT ATTGGCCACT GTCGTTAGTG GACAGAAACA GGATAGACAG GGAGGAGAAC 2100
   GAAGGAGGTC CCAACTCGAT CGCGACCAGT GTGCCTACTG CAAAGAAAAG GGGCACTGGG 2160
   CTAAAGATTG TCCCAAGAAA CCACGAGGAC CTCGGGGACC AAGACCCCAG ACCTCCCTCC 2220
   TGACCCTAGA TGACTAGGGA GGTCAGGGTC AGGAGCCCCC CCCTGAACCC AGGATAACCC 2280
   TCAAAGTCGG GGGGCAACCC GTCACCTTCC TGGTAGATAC TGGGGCCCAA CACTCCGTGC 2340
   TGACCCAAAA TCCTGGACCC CTAAGTGATA AGTCTGCCTG GGTCCAAGGG GCTACTGGAG 2400
   GAAAGCGGTA TCGCTGGACC ACGGATCGCA AAGTACATCT AGCTACCGGT AAGGTCACCC 2460
   ACTCTTTCCT CCATGTACCA GACTGTCCCT ATCCTCTGTT AGGAAGAGAT TTGCTGACTA 2520
   AACTAAAAGC CCAAATCCAC TTTGAGGGAT CAGGAGCTCA GGTTATGGGA CCAATGGGGC 2580
   AGCCCCTGCA AGTGTTGACC CTAAATATAG AAGATGAGCA TCGGCTACAT GAGACCTCAA 2640
   AAGAGCCAGA TGTTTCTCTA GGGTCCACAT GGCTGTCTGA TTTTCCTCAG GCCTGGGCGG 2700
   AAACCGGGGG CATGGGACTG GCAGTTCGCC AAGCTCCTCT GATCATACCT CTGAAAGCAA 2760
   CCTCTACCCC CGTGTCCATA AAACAATACC CCATGTCACA AGAAGCCAGA CTGGGGATCA 2820
   AGCCCCACAT ACAGAGACTG TTGGACCAGG GAATACTGGT ACCCTGCCAG TCCCCCTGGA 2880
   ACACGCCCCT GCTACCCGTT AAGAAACCAG GGACTAATGA TTATAGGCCT GTCCAGGATC 2940
   TGAGAGAAGT CAACAAGCGG GTGGAAGACA TCCACCCCAC CGTGCCCAAC CCTTACAACC 3000
   TCTTGAGCGG GCTCCCACCG TCCCACCAGT GGTACACTGT GCTTGATTTA AAGGATGCCT 3060
   TTTTCTGCCT GAGACTCCAC CCCACCAGTC AGCCTCTCTT CGCCTTTGAG TGGAGAGATC 3120
   CAGAGATGGG AATCTCAGGA CAATTGACCT GGACCAGACT CCCACAGGGT TTCAAAAACA 3180
   GTCCCACCCT GTTTGATGAG GCACTGCACA GAGACCTAGC AGACTTCCGG ATCCAGCACC 3240
   CAGACTTGAT CCTGCTACAG TACGTGGATG ACTTACTGCT GGCCGCCACT TCTGAGCTAG 3300
   ACTGCCAACA AGGTACTCGG GCCCTGTTAC AAACCCTAGG GAACCTCGGG TATCGGGCCT 3360
   CGGCCAAGAA AGCCCAAATT TGCCAGAAAC AGGTCAAGTA TCTGGGGTAT CTTCTAAAAG 3420
   AGGGTCAGAG ATGGCTGACT GAGGCCAGAA AAGAGACTGT GATGGGGCAG CCTACTCCGA 3480
   AGACCCCTCG ACAACTAAGG GAGTTCCTAG GGACGGCAGG CTTCTGTCGC CTCTGGATCC 3540
   CTGGGTTTGC AGAAATGGCA GCCCCCTTGT ACCCTCTCAC CAAAACGGGG ACTCTGTTTA 3600
   ATTGGGGCCC AGACCAACAA AAGGCCTATC AAGAAATCAA GCAAGCTCTT CTAACTGCCC 3660
   CAGCCCTGGG GTTGCCAGAT TTGACTAAGC CCTTTGAACT CTTTGTCGAC GAGAAGCAGG 3720
   GCTACGCCAA AGGTGTCCTA ACGCAAAAAC TGGGACCTTG GCGTCGGCCG GTGGCCTACC 3780
   TGTCCAAAAA GCTAGACCCA GTAGCAGCTG GGTGGCCCCC TTGCCTACGG ATGGTAGCAG 3840
   CCATTGCCGT ACTGACAAAG GATGCAGGCA AGCTAACCAT GGGACAGCCA CTAGTCATTC 3900
   TGGCCCCCCA TGCAGTAGAG GCACTAGTCA AACAACCCCC CGACCGCTGG CTTTCCAACG 3960
   CCCGGATGAC TCACTATCAG GCCTTGCTTT TGGACACGGA CCGGGTCCAG TTCGGACCGG 4020
   TGGTAGCCCT GAACCCGGCT ACGCTGCTCC CACTGCCTGA GGAAGGGCTG CAACACAACT 4080
   GCCTTGATAT CCTGGCCGAA GCCCACGGAA CCCGACCCGA CCTAACGGAC CAGCCGCTCC 4140
   CAGACGCCGA CCACACCTGG TACACGGATG GAAGCAGTCT CTTACAAGAG GGACAGCGTA 4200
   AGGCGGGAGC TGCGGTGACC ACCGAGACCG AGGTAATCTG GGCTAAAGCC CTGCCAGCCG 4260
   GGACATCCGC TCAGCGGGCT GAACTGATAG CACTCACCCA GGCCCTAAAG ATGGCAGAAG 4320
   GTAAGAAGCT AAATGTTTAT ACTGATAGCC GTTATGCTTT TGCTACTGCC CATATCCATG 4380
   GAGAAATATA CAGAAGGCGT GGGTTGCTCA CATCAGAAGG CAAAGAGATC AAAAATAAAG 4440
   ACGAGATCTT GGCCCTACTA AAAGCCCTCT TTCTGCCCAA AAGACTTAGC ATAATCCATT 4500
   GTCCAGGACA TCAAAAGGGA CACAGCGCCG AGGCTAGAGG CAACCGGATG GCTGACCAAG 4560
   CGGCCCGAAA GGCAGCCATC ACAGAGACTC CAGACACCTC TACCCTCCTC ATAGAAAATT 4620
   CATCACCCTA CACCTCAGAA CATTTTCATT ACACAGTGAC TGATATAAAG GACCTAACCA 4680
   AGTTGGGGGC CATTTATGAT AAAACAAAGA AGTATTGGGT CTACCAAGGA AAACCTGTGA 4740
   TGCCTGACCA GTTTACTTTT GAATTATTAG ACTTTCTTCA TCAGCTGACT CACCTCAGCT 4800
   TCTCAAAAAT GAAGGCTCTC CTAGAGAGAA GCCACAGTCC CTACTACATG CTGAACCGGG 4860
   ATCGAACACT CAAAAATATC ACTGAGACCT GCAAAGCTTG TGCACAAGTC AACGCCAGCA 4920
   AGTCTGCCGT TAAACAGGGA ACTAGGGTCC GCGGGCATCG GCCCGGCACT CATTGGGAGA 4980
   TCGATTTCAC CGAGATAAAG CCCGGATTGT ATGGCTATAA ATATCTTCTA GTTTTTATAG 5040
   ATACCTTTTC TGGCTGGATA GAAGCCTTCC CAACCAAGAA AGAAACCGCC AAGGTCGTAA 5100
   CCAAGAAGCT ACTAGAGGAG ATCTTCCCCA GGTTCGGCAT GCCTCAGGTA TTGGGAACTG 5160
   ACAATGGGCC TGCCTTCGTC TCCAAGGTGA GTCAGACAGT GGCCGATCTG TTGGGGATTG 5220
   ATTGGAAATT ACATTGTGCA TACAGACCCC AAAGCTCAGG CCAGGTAGAA AGAATGAATA 5280
   GAACCATCAA GGAGACTTTA ACTAAATTAA CGCTTGCAAC TGGCTCTAGA GACTGGGTGC 5340
   TCCTACTCCC CTTAGCCCTG TACCGAGCCC GCAACACGCC GGGCCCCCAT GGCCTCACCC 5400
   CATATGAGAT CTTATATGGG GCACCCCCGC CCCTTGTAAA CTTCCCTGAC CCTGACATGA 5460
   CAAGAGTTAC TAACAGCCCC TCTCTCCAAG CTCACTTACA GGCTCTCTAC TTAGTCCAGC 5520
   ACGAAGTCTG GAGACCTCTG GCGGCAGCCT ACCAAGAACA ACTGGACCGA CCGGTGGTAC 5580
   CTCACCCTTA CCGAGTCGGC GACACAGTGT GGGTCCGCCG ACACCAGACT AAGAACCTAG 5640
   AACCTCGCTG GAAAGGACCT TACACAGTCC TGCTGACCAC CCCCACCGCC CTCAAAGTAG 5700
   ACGGCATCGC AGCTTGGATA CACGCCGCCC ACGTGAAGGC TGCCGACCCC GGGGGTGGAC 5760
   CATCCTCTAG ACTGACATGG CGCGTTCAAC GCTCTCAAAA CCCCTTAAAA ATAAGGTTAA 5820
   CCCGCGAGGC CCCCTAATCC CCTTAATTCT TCTGATGCTC AGAGGGGTCA GTACTGCTTC 5880
   GCCCGGCTCC AGTCCTCATC AAGTCTATAA TATCACCTGG GAGGTAACCA ATGGAGATCG 5940
   GGAGACGGTA TGGGCAACTT CTGGCAACCA CCCTCTGTGG ACCTGGTGGC CTGACCTTAC 6000
   CCCAGATTTA TGTATGTTAG CCCACCATGG ACCATCTTAT TGGGGGCTAG AATATCAATC 6060
   CCCTTTTTCT TCTCCCCCGG GGCCCCCTTG TTGCTCAGGG GGCAGCAGCC CAGGCTGTTC 6120
   CAGAGACTGC GAAGAACCTT TAACCTCCCT CACCCCTCGG TGCAACACTG CCTGGAACAG 6180
   ACTCAAGCTA GACCAGACAA CTCATAAATC AAATGAGGGA TTTTATGTTT GCCCCGGGCC 6240
   CCACCGCCCC CGAGAATCCA AGTCATGTGG GGGTCCAGAC TCCTTCTACT GTGCCTATTG 6300
   GGGCTGTGAG ACAACCGGTA GAGCTTACTG GAAGCCCTCC TCATCATGGG ATTTCATCAC 6360
   AGTAAACAAC AATCTCACCT CTGACCAGGC TGTCCAGGTA TGCAAAGATA ATAAGTGGTG 6420
   CAACCCCTTA GTTATTCGGT TTACAGACGC CGGGAGACGG GTTACTTCCT GGACCACAGG 6480
   ACATTACTGG GGCTTACGTT TGTATGTCTC CGGACAAGAT CCAGGGCTTA CATTTGGGAT 6540
   CCGACTCAGA TACCAAAATC TAGGACCCCG CGTCCCAATA GGGCCAAACC CCGTTCTGGC 6600
   AGACCAACAG CCACTCTCCA AGCCCAAACC TGTTAAGTCG CCTTCAGTCA CCAAACCACC 6660
   CAGTGGGACT CCTCTCTCCC CTACCCAACT TCCACCGGCG GGAACGGAAA ATAGGCTGCT 6720
   AAACTTAGTA GACGGAGCCT ACCAAGCCCT CAACCTCACC AGTCCTGACA AAACCCAAGA 6780
   GTGCTGGTTG TGTCTAGTAG CGGGACCCCC CTACTACGAA GGGGTTGCCG TCCTGGGTAC 6840
   CTACTCCAAC CATACCTCTG CTCCAGCCAA CTGCTCCGTG GCCTCCCAAC ACAAGTTGAC 6900
   CCTGTCCGAA GTGACCGGAC AGGGACTCTG CATAGGAGCA GTTCCCAAAA CACATCAGGC 6960
   CCTATGTAAT ACCACCCAGA CAAGCAGTCG AGGGTCCTAT TATCTAGTTG CCCCTACAGG 7020
   TACCATGTGG GCTTGTAGTA CCGGGCTTAC TCCATGCATC TCCACCACCA TACTGAACCT 7080
   TACCACTGAT TATTGTGTTC TTGTCGAACT CTGGCCAAGA GTCACCTATC ATTCCCCCAG 7140
   CTATGTTTAC GGCCTGTTTG AGAGATCCAA CCGACACAAA AGAGAACCGG TGTCGTTAAC 7200
   CCTGGCCCTA TTATTGGGTG GACTAACCAT GGGGGGAATT GCCGCTGGAA TAGGAACAGG 7260
   GACTACTGCT CTAATGGCCA CTCAGCAATT CCAGCAGCTC CAAGCCGCAG TACAGGATGA 7320
   TCTCAGGGAG GTTGAAAAAT CAATCTCTAA CCTAGAAAAG TCTCTCACTT CCCTGTCTGA 7380
   AGTTGTCCTA CAGAATCGAA GGGGCCTAGA CTTGTTATTT CTAAAAGAAG GAGGGCTGTG 7440
   TGCTGCTCTA AAAGAAGAAT GTTGCTTCTA TGCGGACCAC ACAGGACTAG TGAGAGACAG 7500
   CATGGCCAAA TTGAGAGAGA GGCTTAATCA GAGACAGAAA CTGTTTGAGT CAACTCAAGG 7560
   ATGGTTTGAG GGACTGTTTA ACAGATCCCC TTGGTTTACC ACCTTGATAT CTACCATTAT 7620
   GGGACCCCTC ATTGTACTCC TAATGATTTT GCTCTTCGGA CCCTGCATTC TTAATCGATT 7680
   AGTCCAATTT GTTAAAGACA GGATATCAGT GGTCCAGGCT CTAGTTTTGA CTCAACAATA 7740
   TCACCAGCTG AAGCCTATAG AGTACGAGCC ATAGATAAAA TAAAAGATTT TATTTAGTCT 7800
   CCAGAAAAAG GGGGGAATGA AAGACCCCAC CTGTAGGTTT GGCAAGCTAG CTTAAGTAAC 7860
   GCCATTTTGC AAGGCATGGA AAAATACATA ACTGAGAATA GAGAAGTTCA GATCAAGGTC 7920
   AGGAACAGAT GGAACAGCTG AATATGGGCC AAACAGGATA TCTGTGGTAA GCAGTTCCTG 7980
   CCCCGGCTCA GGGCCAAGAA CAGATGGAAC AGCTGAATAT GGGCCAAACA GGATATCTGT 8040
   GGTAAGCAGT TCCTGCCCCG GCTCAGGGCC AAGAACAGAT GGTCCCCAGA TGCGGTCCAG 8100
   CCCTCAGCAG TTTCTAGAGA ACCATCAGAT GTTTCCAGGG TGCCCCAAGG ACCTGAAATG 8160
   ACCCTGTGCC TTATTTGAAC TAACCAATCA GTTCGCTTCT CGCTTCTGTT CGCGCGCTTC 8220
   TGCTCCCCGA GCTCAATAAA AGAGCCCACA ACCCCTCACT CGGGGCGCCA GTCCTCCGAT 8280
   TGACTGAGTC GCCCGGGTAC CCGTGTATCC AATAAACCCT CTTGCAGTTG CA 8332
(2) INFORMATION FOR SEQ ID NO:2:
(i) SEQUENCE CHARACTERISTICS:
   (A) LENGTH: 36 base pairs
   (B) TYPE: nucleic acid
   (C) STRANDEDNESS: single
   (D) TOPOLOGY: linear
   (xi) SEQUENCE DESCRIPTION: SEQ ID NO:2:
      GGTAACAGTC TGGCCCGAAT TCTCAGACAA ATACAG 36
2) INFORMATION FOR SEQ ID NO:3:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (xi) SEQUENCE DESCRIPTION: SEQ ID NO:3:
      CTGTATTTGT CTGAGAATTA AGGCTAGACT GTTACCAC 38
(2) INFORMATION FOR SEQ ID NO:4:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:4:
      ATATATATAT ATCGATACCA TG 22
(2) INFORMATION FOR SEQ ID NO:5:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (xi) SEQUENCE DESCRIPTION: SEQ ID NO:5:
      GGCGCCAAAC CTAAAC 16
(2) INFORMATION FOR SEQ ID NO:6:
   (i) SEQUENCE CHARACTERISTICS:
      (A) LENGTH: 5 amino acids
      (B) TYPE: amino acid
      (C) STRANDEDNESS: single (D) TOPOLOGY: linear
   (xi) SEQUENCE DESCRIPTION: SEQ ID NO:6:
      Ser Lys Asn Tyr Pro 5
(2) INFORMATION FOR SEQ ID NO:7:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:7:
      ACCATCCTCT GGACGGACAT G 21
(2) INFORMATION FOR SEQ ID NO:8:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:8:
      ACCCGGCCGT GGACGGACAT G 21
(2) INFORMATION FOR SEQ ID NO:9:
   (i) SEQUENCE CHARACTERISTICS:
      (A) LENGTH: 449 base pairs
      (B) TYPE: nucleic acid
      (C) STRANDEDNESS: single
      (D) TOPOLOGY: linear
   (ix) FEATURE:
      (A) NAME/KEY: CDS
      (B) LOCATION: 20..442
   (xi) SEQUENCE DESCRIPTION: SEQ ID NO:9:
   ATATATATAT ATCGATACC ATG GGG CAA ACC GTG ACT ACC CCT CTG TCC CTC     52
                        Met Gly Gln Thr Val Thr Thr Pro Leu Ser Leu
                         1               5                   10
   ACA CTG GGC CAT TGG AAG GAC GTG GAA AGA ATT GCC CAT AAT CAA AGC     100
   Thr Leu Gly His Trp Lys Asp Val Glu Arg Ile Ala His Asn Gln Ser
               15                  20                  25
   GTG GAC GTC AAA AAA CGC AGG TGG GTG ACA TTT TGT AGC GCC GAG TGG     148
   Val Asp Val Lys Lys Arg Arg Trp Val Thr Phe Cys Ser Ala Glu Trp
           30                  35                  40
   CCC ACA TTC AAT GTT GGC TGG CCT AGG GAT GGA ACT TTC AAT CGC GAT     196
   Pro Thr Phe Asn Val Gly Trp Pro Arg Asp Gly Thr Phe Asn Arg Asp
       45                  50                  55
   CTG ATT ACT CAA GTG AAA ATT AAA GTG TTC AGC CCC GGA CCC CAC GGC     244
   Leu Ile Thr Gln Val Lys Ile Lys Val Phe Ser Pro Gly Pro His Gly
    60                  65                  70                  75
   CAT CCC GAT CAA GTT CCT TAT ATT GTC ACA TGG GAG GCT CTC GCT TTC     292
   His Pro Asp Gln Val Pro Tyr Ile Val Thr Trp Glu Ala Leu Ala Phe
                   80                  85                  90
   GAT CCA CCA CCT TGG GTG AAA CCA TTC GTG CAT CCC AAA CCA CCT CCA     340
   Asp Pro Pro Pro Trp Val Lys Pro Phe Val His Pro Lys Pro Pro Pro
               95                 100                 105
   CCC CTC CCA CCC AGC GCT CCT AGC CTG CCC TTG GAG CCC CCA CGA AGC     388
   Pro Leu Pro Pro Ser Ala Pro Ser Leu Pro Leu Glu Pro Pro Arg Ser
           110                 115                 120
   ACA CCA CCC AGG AGC AGC TTG TAC CCT GCT CTG ACC CCC AGC CTC GGC     436
   Thr Pro Pro Arg Ser Ser Leu Tyr Pro Ala Leu Thr Pro Ser Leu Gly
       125                 130                 135
   GCC AAA CCTAAAC                                                      449
   Ala Lys
   140
(2) INFORMATION FOR SEQ ID NO:10:
   (i) SEQUENCE CHARACTERISTICS:
      (A) LENGTH: 141 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Met Gly Gln Thr Val Thr Thr Pro Leu Ser Leu Thr Leu Gly His Trp
    1               5                  10                  15
   Lys Asp Val Glu Arg Ile Ala His Asn Gln Ser Val Asp Val Lys Lys
               20                  25                  30
   Arg Arg Trp Val Thr Phe Cys Ser Ala Glu Trp Pro Thr Phe Asn Val
           35                  40                  45
   Gly Trp Pro Arg Asp Gly Thr Phe Asn Arg Asp Leu Ile Thr Gln Val
       50                  55                  60
   Lys Ile Lys Val Phe Ser Pro Gly Pro His Gly His Pro Asp Gln Val
   65                  70                  75                  80
   Pro Tyr Ile Val Thr Trp Glu Ala Leu Ala Phe Asp Pro Pro Pro Trp
                   85                  90                  95
   Val Lys Pro Phe Val His Pro Lys Pro Pro Pro Pro Leu Pro Pro Ser
               100                 105                 110
   Ala Pro Ser Leu Pro Leu Glu Pro Pro Arg Ser Thr Pro Pro Arg Ser
           115                 120                 125
   Ser Leu Tyr Pro Ala Leu Thr Pro Ser Leu Gly Ala Lys
       130                 135                 140
(2) INFORMATION FOR SEQ ID NO:11:
   (i) SEQUENCE CHARACTERISTICS:
      (A) LENGTH: 420 base pairs
      (B) TYPE: nucleic acid
      (C) STRANDEDNESS: single
      (D) TOPOLOGY: linear
   (ix) FEATURE:
      (A) NAME/KEY: CDS
      (B) LOCATION: 1..420
   (xi) SEQUENCE DESCRIPTION: SEQ ID NO:11:
   ATG GGC CAG ACT GTT ACC ACT CCC TTA AGT TTG ACC TTA GGT CAC TGG      48
   Met Gly Gln Thr Val Thr Thr Pro Leu Ser Leu Thr Leu Gly His Trp
    1               5                  10                  15
   AAA GAT GTC GAG CGG ATC GCT CAC AAC CAG TCG GTA GAT GTC AAG AAG      96
   Lys Asp Val Glu Arg Ile Ala His Asn Gln Ser Val Asp Val Lys Lys
               20                  25                  30
   AGA CGT TGG GTT ACC TTC TGC TCT GCA GAA TGG CCA ACC TTT AAC GTC     144
   Arg Arg Trp Val Thr Phe Cys Ser Ala Glu Trp Pro Thr Phe Asn Val
           35                  40                  45
   GGA TGG CCG CGA GAC GGC ACC TTT AAC CGA GAC CTC ATC ACC CAG GTT     192
   Gly Trp Pro Arg Asp Gly Thr Phe Asn Arg Asp Leu Ile Thr Gln Val
       50                  55                  60
   AAG ATC AAG GTC TTT TCA CCT GGC CCG CAT GGA CAC CCA GAC CAG GTC     240
   Lys Ile Lys Val Phe Ser Pro Gly Pro His Gly His Pro Asp Gln Val
   65                  70                  75                  80
   CCC TAC ATC GTG ACC TGG GAA GCC TTG GCT TTT GAC CCC CCT CCC TGG        288
   Pro Tyr Ile Val Thr Trp Glu Ala Leu Ala Phe Asp Pro Pro Pro Trp
                    85                  90                  95
   GTC AAG CCC TTT GTA CAC CCT AAG CCT CCG CCT CCT CTT CCT CCA TCC        336
   Val Lys Pro Phe Val His Pro Lys Pro Pro Pro Pro Leu Pro Pro Ser
               100                 105                 110
   GCC CCG TCT CTC CCC CTT GAA CCT CCT CGT TCG ACC CCG CCT CGA TCC        384
   Ala Pro Ser Leu Pro Leu Glu Pro Pro Arg Ser Thr Pro Pro Arg Ser
           115                 120                 125
   TCC CTT TAT CCA GCC CTC ACT CCT TCT CTA GGC GCC                        420
   Ser Leu Tyr Pro Ala Leu Thr Pro Ser Leu Gly Ala
       130                 135                 140
(2) INFORMATION FOR SEQ ID NO:12:
   (i) SEQUENCE CHARACTERISTICS:
      (A) LENGTH: 140 amino acids
      (B) TYPE: amino acid
      (D) TOPOLOGY: linear
   (ii) MOLECULE TYPE: protein
   (xi) SEQUENCE DESCRIPTION: SEQ ID NO:12:
   Met Gly Gln Thr Val Thr Thr Pro Leu Ser Leu Thr Leu Gly His Trp
    1               5                  10                  15
   Lys Asp Val Glu Arg Ile Ala His Asn Gln Ser Val Asp Val Lys Lys
               20                  25                  30
   Arg Arg Trp Val Thr Phe Cys Ser Ala Glu Trp Pro Thr Phe Asn Val
           35                  40                  45
   Gly Trp Pro Arg Asp Gly Thr Phe Asn Arg Asp Leu Ile Thr Gln Val
       50                  55                  60
   Lys Ile Lys Val Phe Ser Pro Gly Pro His Gly His Pro Asp Gln Val
   65                  70                  75                  80
   Pro Tyr Ile Val Thr Trp Glu Ala Leu Ala Phe Asp Pro Pro Pro Trp
                   85                  90                  95
   Val Lys Pro Phe Val His Pro Lys Pro Pro Pro Pro Leu Pro Pro Ser
               100                 105                 110
   Ala Pro Ser Leu Pro Leu Glu Pro Pro Arg Ser Thr Pro Pro Arg Ser
           115                 120                 125
   Ser Leu Tyr Pro Ala Leu Thr Pro Ser Leu Gly Ala
       130                 135                 140
(2) INFORMATION FOR SEQ 10 NO:13:
   (i) SEQUENCE CHARACTERISTICS:
      (A) LENGTH: 2001 base pairs
      (B) TYPE: nucleic acid
      (C) STRANDEDNESS: single
      (D) TOPOLOGY: linear
   (xi) SEQUENCE DESCRIPTION: SEQ ID NO:13:
(2) INFORMATION FOR SEQ ID NO:14:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (xi) SEQUENCE DESCRIPTION: SEQ ID NO:14:
      CTAGCTAGCT AG 12
(2) INFORMATION FOR SEQ ID NO:15:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (xi) SEQUENCE DESCRIPTION: SEQ ID NO:15:
   ATATATATAT ATCGATACCA TGGGGCAAAC CGTGACTACC CCTCTGTCCC TCACACTGGC 60
   CCAA 64
(2) INFORMATION FOR SEQ ID NO:16:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:16:
      TTGATTATGG GCAATTCTTT CCACGTCCTT CCAATGGCCC AGTGTGAGGG AC 52
(2) INFORMATION FOR SEQ ID NO:17:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (xi) SEQUENCE DESCRIPTION: SEQ ID NO:17:
   AGAATTGCCC ATAATCAAAG CGTGGACGTC AAAAAACGCA GGTGGGTGAC ATTTTGTAGC 60
   GCCGAGTGGC CC 72
(2) INFORMATION FOR SEQ ID NO:18:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:18:
      AAGTTCCATC CCTAGGCCAG CCAACATTGA ATGTGGGCCA CTCGGCGCTA CA 52
(2) INFORMATION FOR SEQ ID NO:19:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (xi) SEQUENCE DESCRIPTION: SEQ ID NO:19:
   GGCCTAGGGA TGGAACTTTC AATCGCGATC TGATTACTCA AGTGAAAATT AAAGTGTTCA 60
   GCCCCGGACC CC 72
(2) INFORMATION FOR SEQ ID NO:20:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:20:
      GTGACAATAT AAGGAACTTG ATCGGGATGG CCGTGGGGTC CGGGGCTGAA CA 52
(2) INFORMATION FOR SEQ ID NO:21:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (xi) SEQUENCE DESCRIPTION: SEQ ID NO:21:
   AGTTCCTTAT ATTGTCACAT CGGAGGCTCT CGCTTTCGAT CCACCACCTT GGGTGAAACC 60
   ATTCGTGCAT CC 72
(2) INFORMATION FOR SEQ ID NO:22:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:22
      AGGAGCGCTG GGTGGGAGGG GTGGAGGTGG TTTGGGATGC ACGAATGGTT TC 52
(2) INFORMATION FOR SEQ ID NO:23
   (i) SEQUENCE CHARACTERISTICS:
      (A) LENGTH: 72 base pairs
      (B) TYPE: nucleic acid (C) STRANDEDNESS: single
      (D) TOPOLOGY: linear
   (xi) SEQUENCE DESCRIPTION: SEQ ID NO:23:
   CTCCCACCCA GCGCTCCTAG CCTGCCCTTG GAGCCCCCAC GAAGCACACC ACCCAGGAGC 60
   AGCTTGTACC CT 72
(2) INFORMATION FOR SEQ ID NO:24:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:24:
      GTTTAGGTTT GGCGCCGAGG CTGGGGGTCA GAGCAGGGTA CAAGCTGCTC CT 52
(2) INFORMATION FOR SEQ ID NO:25:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (xi) SEQUENCE DESCRIPTION: SEQ ID NO:25:
      ATATATATAT ATCGATACC 19
(2) INFORMATION FOR SEQ ID NO:26:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:26:
      GTTTAGGTTT GGCGCCGAGG 20